# FASCICULE DE BREVET EUROPEEN

(11) **EP 1 523 498 B1**
(45) Date de publication et mention de la délivrance du brevet: **28.09.2011**
(21) Numéro de dépôt: 03760733.0
(22) Date de dépôt: 18.06.2003
(51) Int. Cl.: C07K 14/36, C12P 21/04, C12N 15/68, C12Q 1/68

(54) **POLYNUCLEOTIDES ET POLYPEPTIDES CODES PAR LESDITS POLYNUCLEOTIDES IMPLIQUES DANS LA SYNTHESE DE DERIVES DES DICETOPIPERAZINES**
AN DER SYNTHESE VON DERIVATEN DES DIKETOPIPERAZINS BETEILIGTE POLYPEPTIDE UND POYNUKLEOTIDE, DIE FÜR DIESE KODIEREN
POLYNUCLEOTIDES AND POLYPEPTIDES CODED BY SAID POLYNUCLEOTIDES INVOLVED IN THE SYNTHESIS OF DIKETOPIPERAZINE DERIVATIVES

(30) Priorité: 21.06.2002 FR 0207728
(43) Date de publication de la demande: 20.04.2005
(73) Titulaire: Commissariat à l'Énergie Atomique et aux Énergies Alternatives, 75015 Paris (FR); CENTRE NATIONAL DE LA RECHERCHE SCIENTIFIQUE, 75794 Paris Cedex 16 (FR)
(72) Inventeur: GONDRY, Muriel, F-91470 Limours-en-Hurepoix (FR); GENET, Roger, F-91470 Limours-en-Hurepoix (FR); LAUTRU, Sylvie, F-78480 Verneuil-sur-Seine (FR); PERNODET, Jean-Luc, F-94230 Cachan (FR)
(74) Mandataire: Vialle-Presles, Marie José
(86) Numéro de dépôt international: PCT/FR2003/001851
(87) Numéro de publication internationale: WO 2004/000879

(56) Documents cités:
- GONDRY MURIEL ET AL: "Cyclic dipeptide oxidase from Streptomyces noursei: Isolation, purification and partial characterization of a novel, amino acyl alpha,beta-dehydrogenase." mars 2001 (2001-03) , EUROPEAN JOURNAL OF BIOCHEMISTRY, VOL. 268, NR. 6, PAGE(S) 1712-1721 XP002242439 ISSN: 0014-2956 cité dans la demande
- KANZAKI HIROSHI ET AL: "Enzymatic conversion of cyclic dipeptides to dehydro derivatives that inhibit cell division." JOURNAL OF BIOSCIENCE AND BIOENGINEERING, vol. 90, no. 1, juillet 2000 (2000-07), pages 86-89, XP002242437 ISSN: 1389-1723
- KANZAKI HIROSHI ET AL: "Biosynthetic intermediates of the tetradehydro cyclic dipeptide albonoursin produced by Streptomyces albulus KO-23." JOURNAL OF ANTIBIOTICS (TOKYO), vol. 53, no. 11, novembre 2000 (2000-11), pages 1257-1264, XP001149049 ISSN: 0021-8820
- LAUTRU SYLVIE ET AL: "The albonoursin gene cluster of S. noursei: Biosynthesis of diketopiperazine metabolites independent of nonribosomal peptide synthetases." 20 décembre 2002 (2002-12-20) , CHEMISTRY & BIOLOGY (CAMBRIDGE), VOL. 9, NR. 12, PAGE(S) 1355-1364 XP002242438 ISSN: 1074-5521 le document en entier

## Description

La présente invention est relative à de nouveaux polynucléotides isolés, naturels ou synthétiques, et aux polypeptides codés par lesdits polynucléotides, impliqués dans la synthèse de dérivés des dicétopipérazines, aux vecteurs comprenant lesdits polynucléotides, aux micro-organismes transformés avec lesdits polynucléotides, aux applications desdits polynucléotides et desdits polypeptides, ainsi qu'à des procédés de synthèse de dérivés des dicétopipérazines dont des cyclodipeptides et des dicétopipérazines substituées en positions 3 et 6 par des chaînes latérales d'acides aminés α,β-insaturées.

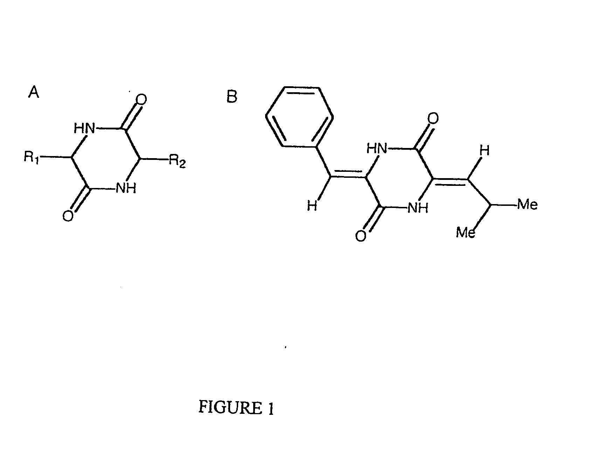

Par dérivés des dicétopipérazines, on entend au sens de la présente invention des molécules ayant un noyau dicétopipérazine (pipérazine-2,5-diones ou 2,5-dioxopipérazines ou 2,5-DKP), substitué en positions 3 et 6 par des acides aminés. Dans le cas particulier des cyclo-diaminoacides (cyclodipeptides ou dipeptides cycliques), les groupes substituants en positions 3 et 6 sont les chaînes latérales d'aminoacides. Dans le cas particulier des cyclo-bi-déshydro-di-aminoacides (cyclo-bisdéshydro-dipeptides), les groupes substituants en positions 3 et 6 sont les chaînes latérales des aminoacides α,β-insaturées (Figure 1).

Les dérivés des dicétopipérazines constituent une famille de composés essentiellement produits par les microorganismes tels que les bactéries, les levures, les champignons filamenteux et les lichens. D'autres ont également été isolés dans des organismes marins tels que les éponges et les étoiles de mer. Un exemple de ces dérivés a été mis en évidence chez l'homme : le cyclo(L-His-L-Pro).

Les dérivés des dicétopipérazines présentent des structures très variées allant des cyclodipeptides simples à des structures beaucoup plus complexes.

Les cyclodipeptides simples ne constituent qu'une faible fraction des dérivés des dicétopipérazines dont la majorité présente des structures plus complexes où le cycle principal et/ou les chaînes latérales comportent de nombreuses modifications : introduction de groupements carbonés, hydroxyle, nitro, époxy, acétyle, ou méthoxy, ainsi que la formation de ponts disulfures ou d'hétérocycles. La formation de double liaison entre deux carbones est également assez répandue. Certains dérivés, d'origine marine, incorporent des atomes d'halogène.

Quelques exemples d'acides aminés incorporés dans les cyclodipeptides sont présentés dans le Tableau I suivant :

**Tableau I**

| Cyclodipeptide | Organisme |
|---|---|
| Cyclo(Gly-L-Pro) | *Luidia clathrata* |
| Cyclo(L-Pro-L-Leu) | *Rosellinia necatrix* |
| Cyclo(L-Ala-L-Val) | *Aspergillus ochraceus* |
| Cyclo(L-ALa-L-Leu) | *Asperpillus niger* |
| Cyclo(D-Ala-N-méthyl-L-Leu) | *Beauveria nivea* |
| Cyclo(L-Pro-L-Val) | *Aspergillus ochraceus* |
| Cyclo(L-Pro-L-Leu) | *Rosellinia necatrix* |
| Cyclo(D-Val-L-Trp) | Aspergillus chevalieri |
| Cyclo(L-Phe-L-Phe) | *Penicillium nigricans* |
| | *Streptomyces noursei* |
| Cyclo(ΔPhe-ΔLeu) (albonoursine) | *Streptomyces noursei* |
| Cyclo(L-Pro-L-Tyr) | *Alternaria alternata* |
| Cyclo(L-Pro-L-Trp) | *Penicillium brevicompactum* |
| Cyclo(L-Ser-L-Ser) | *Streptomyces orchidaceus* |
| Cyclo(L-Arg-D-Pro) | *Pseudomonas sp.* |
| Phénylahistine | |
| Roquefortine | *Penicillium roquefortii* |
| Cyclo(L-Trp-ΔAba) | *Streptomyces spectabilis* |
| Cyclo(4-methyl-D-Pro-L-Nva) | *Calyx cf. podatypa* |
| Cyclo(ΔAla-L-Val) | *Pseudomonas aerupinosa* |

Peu de choses sont connues quant au rôle physiologique des dérivés des dicétopipérazines. Il a été décrit que le cyclo(ΔAla-L-Val) produit par *Pseudomonas aeruginosa* pourrait être impliqué dans les signaux de communication interbactériens. D'autres composés sont décrits comme impliqués dans la virulence de microorganismes pathogènes ou encore comme se liant au fer ou comme possédant des propriétés neurobiologiques.

Les dérivés des dicétopipérazines se sont avérés intéressants depuis qu'il a été découvert pour certains d'entre eux des propriétés biologiques telles que par exemple des activités anti-bactériennes, antifongiques, antivirales, immunosuppressives ou encore anti-tumorales.

Le Tableau II suivant présente quelques exemples de dérivés des dicétopipérazines ayant une activité biologique connue :

**Tableau II**

| Molécules | Organisme | Activité |
|---|---|---|
| Ambewelamides A et B | *Usnea sp.* | Cytotoxicité |
| Aranotine | *Arachniotus aureus* | Antiviral |
| Bicyclomycine | *Streptomyces sapporonensis* | Antibactérien |
| | | (Inhibition de la terminaison de la transcription) |
| Cyclo(Δ-Ala-L-Leu) | *Penicillium sp.* (F70614) | Inhibition de l'α-glucosidase |
| Cyclo(N-methyl-Tyr)₂ | *Streptomyces griseus* | Inhibition de la calpaine |
| Cyclo(Trp-Δ-Aba) | *Streptomyces spectabilis* | Inhibtion de la gluthation-S-transférase |
| Gliotoxine | *Aspergillus flavus* | Herbicide, antifongique, antibactérien, antiviral |
| Haematocine | *Nectria haematococca* | Antifongique |
| Hyalodendrine | *Penicillium turbatum* | Antibiotique |
| Mycélianamide | Penicillium sp. | Antibactérien |
| | | (Inhibition de la butylcholinestérase) |
| Phénylahistine | *Aspergillus ustus* (NSC-FO38 | Inhibition de la polymérisation des microtubules |
| Tan-1496 A, C et E | *Microphaeropsis sp.* (FL-16144) | Inhibition de topoisomérase |
| | | Antibactérien (Gram +) |
| Verticilline A | *Gliocladium sp.* (SCF-1168) | Inhibition de l'induction du protooncogène c-fos |
| XR334 | *Streptomyces sp.* (X01/4/100) | Inhibition du PAI-I |

Bien que l'étude de ces molécules se soit largement développée, peu de choses sont connues en ce qui concerne leur synthèse. On sait généralement que, chez les bactéries et chez les champignons, ces molécules sont produites par biosynthèse non-ribosomique. Dans certains cas, il a pu être montré que la formation du cycle dicétopipérazine se produit dans des molécules qui sont au préalable activées via une liaison thioester à une enzyme et pour lesquelles la conformation en cis de la liaison peptidique, nécessaire à la réaction de cyclisation, est favorisée par la présence de résidus proline. Dans d'autre cas, il a été mis en évidence que la N-alkylation, particulièrement la N-méthylation, des résidus d'acides aminés favorise également la conformation en cis de la liaison peptidique.

Ainsi toutes les études menées jusqu'à présent ont démontré que la structure primaire de la molécule précurseur, conditionnant sa conformation, est fondamentale pour que la formation du cycle dicétopipérazine se réalise et que le processus aboutisse à la production du dérivé de dicétopipérazine final.

Mais il existe des dérivés des dicétopipérazines qui ne contiennent pas de résidu proline ni de résidu N-alkylé. A titre d'exemple de tels dérivés, on peut citer l'albonoursine ou cyclo(ΔPhe-ΔLeu), antibiotique produit par *Streptomyces noursei*. Il est connu qu'il existe dans *Streptomyces noursei* une activité enzymatique qui catalyse la dernière étape de la production de l'albonoursine, à savoir la formation des résidus α,β-insaturés (GONDRY et Col., Eur. J. Biochem., 2001, 268, 1712-1721). Toutefois, cette activité enzymatique nécessite un substrat sous forme cyclique, le cyclo(L-Phe-L-Leu), qui ne contient pas de résidu proline ni de résidu N-alkylé et dont la voie de synthèse est inconnue.

Ainsi, les dérivés des dicétopipérazines présentent-ils une très grande diversité de structure et des activités biologiques très variées qui en font des molécules intéressantes pour la découverte et la mise au point de nouveaux médicaments.

Pour ce faire, il est nécessaire de pouvoir disposer de grandes quantités de ces molécules.

Certes des voies de synthèse chimique de dérivés des dicétopipérazines ont été décrites, mais pour les dérivés les plus complexes, les rendements sont faibles et les procédés ne sont pas toujours industrialisables.

Comprendre les voies de synthèse naturelle des dérivés des dicétopipérazines, particulièrement celle des cyclodipeptides, pourrait permettre l'amélioration' génétique raisonnée des organismes producteurs, et ouvrirait des perspectives pour substituer ou améliorer les procédés de synthèse existants (par des voies chimiques ou biotechnologiques) grâce à l'optimisation des rendements de production et de purification. En outre, la modification de la nature et/ou de la spécificité des enzymes impliquées dans la voie de biosynthèse des dérivés des dicétopipérazines pourrait conduire à la création de nouveaux dérivés aux structures moléculaires originales et aux propriétés biologiques optimisées.

C'est dans ce cadre que se situe la présente invention.

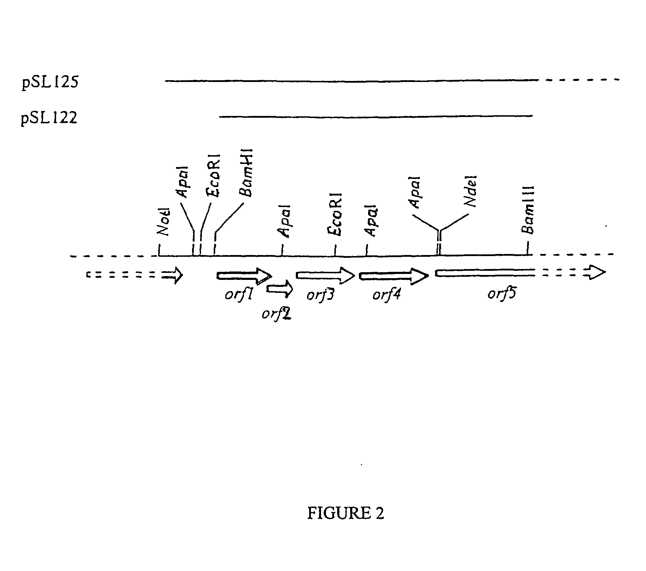

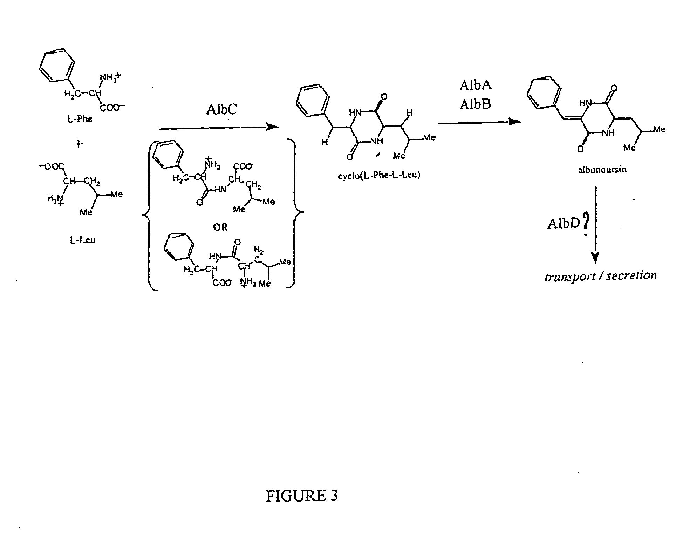

En étudiant la voie de synthèse de l'albonoursine, les Inventeurs ont mis en évidence un polynucléotide (ci-après dénommé polynucléotide BamH1 (SEQ ID N°5)), comprenant quatre phases ouvertes de lecture codant chacune pour un polypeptide responsable de chacune des étapes de la synthèse et du transport de l'albonoursine à partir de résidus L-phénylalanine et L-leucine chez *Streptomyces noursei* et chez des hôtes hétérologues comme *Streptomyces lividans* (voir Figures 2 et 3).

Les Inventeurs ont pu montrer que :
- la première phase ouverte de lecture orf1 (albA, SEQ ID N°1) code pour un polypeptide (AlbA, SEQ ID N°6) impliqué dans une activité cyclodipeptide oxydase (CDO) telle que celle décrite dans GONDRY et al., (Eur. J. Biochem., 2001, 268, 1712-1721) (α,β-désaturation).
- la deuxième phase ouverte de lecture orf2 (albB, SEQ ID N°2) code pour un polypeptide qui est traduit sous deux isoformes (AlbB₁, SEQ ID N°7 et AlbB₂, SEQ ID N°8) nécessaires à l'activité du polypeptide AlbA. Les deux isoformes d'AlbB qui sont exprimées en quantité à peu près équivalentes, se différencient par la présence de 5 acides aminés supplémentaires localisés à l'extrémité N-terminale d'AlbB₁ et résultant de l'utilisation de deux codons d'initiation différents. Dans le cas d'AlbB₁, la méthionine initiale est éliminée.
- la troisième phase ouverte de lecture orf3 (albC, SEQ ID N°3) code pour un polypeptide (AlbC, SEQ ID N°9) ne présentant aucune similitude avec une peptide-synthétase et qui est capable de catalyser la condensation de deux résidus d'acides aminés pour former un dipeptide cyclique. Par exemple chez *Streptomyces noursei,* AlbC catalyse la condensation d'une L-phénylalanine et d'une L-leucine ou de deux L-phénylalanines, pour former le dipeptide cyclique cyclo(L-Phe-L-Leu), précurseur nécessaire à la formation de l'albonoursine et le dipeptide cyclique cyclo(L-Phe-L-Phe). Dans ce cas particulier, AlbC catalyse la cyclisation de résidus d'acides aminés qui ne sont ni une proline ni un résidu N-alkylé, et
- la quatrième phase ouverte de lecture orf4 (albD, SEQ ID N°4) code pour un polypeptide (AlbD, SEQ ID N°10) qui n'intervient pas directement dans la succession de réactions aboutissant à partir d'acides aminés à la formation de dérivés des dicétopipérazines α,β-insaturées, mais est probablement impliqué dans le mécanisme de transport desdits dérivés.

Les Inventeurs ont ainsi montré que pour la synthèse de dérivés des dicétopipérazines α,β-insaturées, seules les trois phases ouvertes de lecture albA, albB et albC sont absolument nécessaires, particulièrement pour la synthèse de l'albonoursine chez *Streptomyces noursei*.

Ainsi l'invention a pour objet un polynucléotide isolé, naturel ou synthétique, caractérisé en ce qu'il comprend au moins les trois phases ouvertes de lecture albA, albB et albC correspondant respectivement aux séquences SEQ ID N°1, SEQ ID N°2 et SEQ ID N°3.

Ce polynucléotide code pour les enzymes nécessaires à la synthèse des dérivés dicétopipérazines α,β-insaturées.

Selon un mode de réalisation avantageux de l'invention, ledit polynucléotide comprend en outre la phase ouverte de lecture albD correspondant à la séquence SEQ ID N°4.

Ce polynucléotide code pour les enzymes nécessaires à la synthèse des dérivés des dicétopipérazines α,β-insaturées et à leur transport et à leur sécrétion.

Selon une forme particulière de l'invention, le polynucléotide répond à la séquence SEQ ID N°5. Ce polynucléotide (polynucléotide BamH1) contient les quatre phases ouvertes de lecture albA, albB, albC et albD et code donc pour les enzymes nécessaires à la synthèse des dérivés dicétopipérazines α,β-insaturées et à leur transport et à leur sécrétion.

L'invention a aussi pour objet un polynucléotide isolé, naturel ou synthétique, caractérisé en ce qu'il comprend au moins l'une des trois phases ouvertes de lecture albB, albC et albD correspondant respectivement aux séquences SEQ ID N°2, SEQ ID N°3 et SEQ ID N°4.

Ainsi, le polynucléotide comprenant la phase ouverte de lecture albC (SEQ ID N°3) code pour une enzyme permettant la cyclisation de deux acides aminés, identiques ou différents pour former un dipeptide cyclique. Le polynucléotide comprenant les phases ouvertes de lecture albC et albD (SEQ ID N°3 et SEQ ID N°4) code pour les enzymes permettant d'une part la cyclisation de deux acides aminés, identiques ou différents pour former un dipeptide cyclique et d'autre part le transport dudit dipeptide.

L'invention a également pour objet un polynucléotide isolé, naturel ou synthétique, répondant à l'une quelconque des séquences SEQ ID N°2 (albB, 318 nucléotides), SEQ ID N°3 (albC, 720 nucléotides) ou SEQ ID N°4 (albD, 834 nucléotides).

L'invention a aussi pour objet des fragments des polynucléotides tels que définis ci-dessus. Par fragment, on entend toute séquence d'au moins 15 acides nucléiques.

Le polynucléotide selon l'invention peut être obtenu à partir de banques d'ADN, particulièrement de banques d'ADN de micro-organismes, très particulièrement à partir d'une banque d'ADN de *Streptomyces noursei.* Le polynucléotide de l'invention peut également être obtenu par une réaction de polymérisation en chaîne (PCR) effectuée sur l'ADN total de *Streptomyces noursei*. Les polynucléotides selon l'invention peuvent être obtenus par RT-PCR effectuée sur les ARN totaux de *Streptomyces noursei.*

L'invention a également pour objet un vecteur dans lequel est inséré l'un quelconque des polynucléotides précédemment décrits. Ainsi, le vecteur de l'invention peut comprendre le polynucléotide comprenant les trois ou les quatre phases ouvertes de lecture albA, albB, albC et/ou albD, correspondant respectivement aux séquences SEQ ID N°1, SEQ ID N°2, SEQ ID N°3 et/ou SEQ ID N°4, le polynucléotide comprenant au moins l'une des trois phases ouvertes de lecture albB, albC ou albD correspondant respectivement aux séquences SEQ ID N°2, SEQ ID N°3 et SEQ ID N°4, l'un quelconque des polynucléotides répondant à l'une au moins des trois phases ouvertes de lecture albB, albC ou albD correspondant respectivement aux séquences SEQ ID N°2, SEQ ID N°3 et SEQ ID N°4, le polynucléotide BamH1 répondant à la séquence SEQ ID N°5 ou encore un fragment desdits polynucléotides.

Le vecteur utilisé peut être tout vecteur connu de l'art antérieur. Particulièrement, on peut citer comme vecteurs utilisables selon l'invention, les plasmides, les cosmides, les chromosomes artificiels bactériens (BAC), les éléments intégratifs d'actinobactéries, les virus ou encore les bactériophages.

Ledit vecteur peut comporter en outre toutes séquences régulatrices requises pour la réplication du vecteur et/ou l'expression du polypeptide codé par le polynucléotide (promoteur, sites de terminaison, etc).

L'invention a également pour objet l'utilisation de l'un au moins des polynucléotides tels que définis précédemment ou de l'un de ses fragments comme sonde pour détecter des séquences correspondantes dans d'autres organismes ou comme amorce pour l'amplification de telles séquences.

Lorsqu'il s'agit d'amorces, lesdits polynucléotides ou lesdits fragments incluent également les séquences anti-sens.

Une des utilisations préférentielle des sondes ou amorces précédemment décrites est la recherche de séquences polynucléotidiques homologues aux séquences des phases ouvertes de lecture albA, albB, albC ou albD dans d'autres organismes, afin en particulier de mettre en évidence de nouvelles voies de synthèse de dérivés des dicétopipérazines.

L'invention a également pour objet un polypeptide isolé, naturel ou synthétique, caractérisé en ce qu'il comprend au moins l'une quelconque des séquences SEQ ID N°7 à SEQ ID N°10, correspondant respectivement aux polypeptides AlbB₁, AlbB₂, AlbC ou AlbD.

L'invention a pour objet un polypeptide isolé, naturel ou synthétique, caractérisé en ce qu'il répond à l'une quelconque des séquences SEQ ID N°7 (AlbB₁), SEQ ID N°8 (AlbB₂), SEQ ID N°9 (AlbC) ou SEQ ID N°10 (AlbD).

L'invention concerne également les polypeptides codés par l'un quelconque des polynucléotides de l'invention, particulièrement l'un quelconque des polynucléotides choisi parmi l'une quelconque des séquences SEQ ID N°2 (albB), SEQ ID N°3 (albC) ou SEQ ID N°4 (albD).

De manière avantageuse, les polypeptides selon l'invention peuvent être soit isolés de microorganismes (*Streptomyces noursei* par exemple), soit obtenus par synthèse chimique ou encore par des moyens biotechnologiques, à partir des polynucléotides de l'invention, comme par exemple à partir de microorganismes modifiés, qui n'expriment normalement pas lesdits polypeptides.

L'invention a également pour objet un polypeptide isolé, dont la séquence est substantiellement homologue à l'une au moins des séquences SEQ ID N°7 à SEQ ID N°10, telles que définies ci-dessus.

On considère ici qu'un polypeptide présente une séquence substantiellement homologue lorsque sa séquence en acides aminés présente au moins 80 % de similarité avec la séquence en acides aminés d'au moins l'une des séquences SEQ ID N°7 à SEQ ID N°10 et que le polypeptide a conservé son activité initiale.

Par 80 % de similarité entre un polypeptide P et les séquences SEQ ID N°7 à 10, on entend que lorsque les deux polypeptides sont alignés, 80% des acides aminés de P sont identiques à l'acide aminé correspondant des séquences SEQ ID N°7 à 10 ou sont remplacés par un acide aminé du même groupe.

Par acide aminé de même groupe, on entend un acide aminé possédant des propriétés chimiques sensiblement identiques. En particulier, on entend par ce terme des acides aminés ayant sensiblement la même charge et/ou la même taille, et/ou la même hydrophilie ou hydrophobie et/ou la même aromaticité.

De tels groupes d'acides aminés incluent notamment ;
(i) glycine, alanine
(ii) isoleucine, leucine, valine
(iii) tryptophane, tyrosine, phénylalanine
(iv) acide aspartique, acide glutamique
(v) arginine, lysine, histidine
(vi) sérine, thréonine

D'autres substitutions peuvent être envisagées, dans lesquelles on remplace un acide aminé par un autre acide aminé comparable mais non naturel (hydroxyproline, norleucine, ornithine, citrulline, cyclo-hexylalanine, acides aminés dextrogyres, ....).

L'invention a également pour objet l'utilisation des polynucléotides ou des vecteurs de l'invention tels que décrits précédemment pour la synthèse de polypeptides correspondant aux séquences SEQ ID N°7 à 10.

L'invention a également pour objet l'utilisation, particulièrement *in vitro,* des polypeptides selon l'invention, seuls ou en combinaison, pour la préparation de cyclodipeptides et/ou de dérivés des dicétopipérazines substituées en positions 3 et 6 par des chaînes latérales d'acides aminés α,β-insaturées, particulièrement de l'albonoursine.

L'invention a également pour objet l'utilisation des polypeptides de l'invention, seuls ou en combinaison, pour modifier l'activité pharmacologique d'une molécule biologique en modifiant sa structure, par exemple par déshydrogénation de chaînes latérales, particulièrement de chaînes latérales d'acides aminés ou par cyclisation, particulièrement de molécules peptidiques.

L'invention a aussi pour objet un système biologique modifié dans lequel, au moins un polynucléotide selon l'invention ou au moins un vecteur selon l'invention a été introduit.

Un tel système biologique peut être tout système d'expression hétérologue connu, utilisant comme hôtes des procaryotes ou des eucaryotes. A titre d'exemple on peut citer un microorganisme comme une bactérie telle que *Escherichia coli* ou *Streptomyces lividans* ou des cellules animales ou d'insectes.

L'invention a aussi pour objet un système acellulaire *in vitro* modifié dans lequel, au moins un polynucléotide selon l'invention ou au moins un vecteur selon l'invention a été introduit.

L'invention a également pour objet l'utilisation d'au moins un polynucléotide selon l'invention et/ou d'au moins un vecteur selon l'invention pour la préparation d'un système biologique modifié, celui-ci pouvant être un microorganisme, comme une bactérie telle que *Escherichia coli* ou *Streptomyces lividans* ou tout système d'expression hétérologue connu utilisant comme hôtes des procaryotes ou des eucaryotes, ou encore d'un système acellulaire *in vitro* modifié.

L'introduction du polynucléotide et/ou du vecteur selon l'invention dans le système biologique modifié hôte, peut se faire par toute méthode connue, comme par exemple la transfection, l'infection, la fusion, l'électroporation, la microinjection ou encore la biolistique.

L'invention a aussi pour objet l'utilisation d'au moins un système biologique modifié ou d'un système acellulaire *in vitro* modifié, tels que définis ci-dessus, pour la préparation de cyclodipeptides et/ou de dérivés des dicétopipérazines substituées en positions 3 et 6 par des chaînes latérales d'acides aminés α,β-insaturées, particulièrement de l'albonoursine.

Les systèmes biologiques sont adaptés à la synthèse, avec un bon rendement, des cyclodipeptides et des dérivés des dicétopipérazines α,β-insaturées, tels que définis ci-dessus.

Lorsqu'il s'agit d'un microorganisme, le système biologique modifié peut éventuellement en outre permettre la sécrétion du dérivé des dicétopipérazines selon l'invention dans un milieu de culture rendant son extraction et sa purification plus faciles. La présence de AlbD dans les systèmes biologiques tels que les microorganismes constitue une étape avantageuse pour le procédé industriel en facilitant l'extraction et la purification du dérivé qui est ainsi secrété dans le milieu de culture.

L'invention a également pour objet un procédé de synthèse *in vitro* d'un cyclodipeptide, caractérisé en ce que :
(1) on met en contact, dans des conditions convenables, deux acides aminés, identiques ou différents, et AlbC (SEQ ID N°9) et
(2) on purifie le cyclodipeptide obtenu.

L'invention a aussi pour objet un procédé de synthèse *in vitro* d'un dérivé des dicétopipérazines, substitué en positions 3 et 6 par des chaînes latérales d'acides aminés α,β-insaturées, caractérisé en ce que :
(1) on met en contact, dans des conditions convenables, deux acides aminés, identiques ou différents, avec AlbC (SEQ ID N°9) et
(2) on met en contact le cyclodipeptide obtenu à l'étape (1) avec AlbA (SEQ ID N°6), AlbB1 (SEQ ID N°7) et AlbB2 (SEQ ID N°8), puis l'on purifie le dérivé des dicétopipérazines α,β-insaturé obtenu. Le procédé peut en outre inclure à l'étape (2), le polypeptide AlbD (SEQ ID N°10). Le procédé peut éventuellement comprendre entre l'étape (1) et l'étape (2), une étape supplémentaire de purification du cyclodipeptide obtenu à l'étape (1).

Ce procédé peut, bien entendu, être réalisé en une seule étape dans laquelle on met en contact dans des conditions convenables, deux acides aminés, identiques ou différents avec AlbA (SEQ ID N°6), albB1 (SEQ ID N°7), AlbB2 (SEQ ID N°8) et AlbC (SEQ ID N°9), éventuellement AlbD (SEQ ID N°10), et que l'on purifie le dérivé des dicétopipérazines α,β-insaturé obtenu.

Par conditions convenables, on entend, de préférence, les conditions dans lesquelles on incube :
- les polypeptides (AlbA, AlbB, AlbC et/ou albD) à des concentrations comprises entre 0,1 nM et 10 µM, de préférence entre 10 nM et 1 µM ;
- en présence d'acides aminés identiques ou différents à une concentration comprise entre 0,1 mM et 100 mM, de préférence entre 1 mM et 10 mM ;
- dans un tampon 0,1 M Tris-HCl, ayant un pH compris entre 6,8 et 8,0, à une température comprise entre 28°C et 40°C pendant une durée comprise entre 2 heures et 48 heures.

L'invention a également pour objet un procédé de synthèse d'un cyclodipeptide, caractérisé en ce que :
(1) on met en contact un système biologique comprenant au moins le polynucléotide SEQ ID N°3 (albC, codant pour AlbC), dans des conditions appropriées à la culture dudit système biologique choisi, et
(2) on purifie le cyclodipeptide obtenu. Le système biologique peut en outre comprendre le polynucléotide SEQ ID N°4 (codant pour AlbD).

L'invention a également pour objet un procédé de synthèse d'un dérivé de dicétopipérazine, substitué en positions 3 et 6 par des chaînes latérales d'acides aminés α,β-insaturées, caractérisé en ce que :
(1) on met en contact un système biologique comprenant le polynucléotide correspondant aux séquences SEQ ID N°1 à 3 (codant pour AlbA, AlbB, et AlbC), dans des conditions appropriées à la culture dudit système biologique choisi, et
(2) on purifie le dérivé des dicétopipérazines α,β-insaturé obtenu. Le système biologique peut en outre comprendre le polynucléotide correspondant à la séquence SEQ ID N°4 (codant pour AlbD).

Par conditions appropriées à la culture dudit système biologique choisi, on comprend que le procédé est mis en oeuvre dans les conditions de culture du système biologique choisi incluant un milieu de culture approprié contenant un large excès en acides aminés. Par exemple, si le système biologique est un microorganisme, comme par exemple *Escherichia coli*, les conditions appropriées sont celles couramment utilisées pour la culture de cette bactérie. Il en est de même pour *Streptomyces lividans* ou si le système biologique est une cellule eucaryote.

Selon les procédés de l'invention, les acides aminés, identiques ou différents, sont présents en une quantité comprise entre 0,1 mM et 100 mM, de préférence entre 1 mM et 10 mM.

De même, selon les procédés de l'invention, les polypeptides AlbA, AlbB, AlbC et AlbD sont présents en une quantité comprise entre 0,1 nM et 10 µM, de préférence entre 10 nM et 1 µM.

La purification des cyclodipeptides et des dérivés des dicétopipérazines α,β-insaturés peut être faite directement à partir de synthèses *in vivo* ou *in vitro* par des techniques d'extraction en phase liquide ou par précipitation, ou des techniques de chromatographie en couche mince ou en phase liquide, en particulier l'HPLC en phase inverse ou toute méthode appropriée à la purification des peptides, bien connu de l'homme du métier.

Les procédés de l'invention peuvent être réalisés dans tout système biologique approprié, particulièrement dans un hôte, comme par exemple un microorganisme comme une bactérie telle que *Escherichia coli* ou *Streptomyces lividans* ou tout système d'expression hétérologue connu utilisant comme hôtes des procaryotes ou des eucaryotes, voire des systèmes acellulaires *in vitro*.

Outre les dispositions qui précèdent, l'invention comprend encore d'autres dispositions qui ressortiront de la description qui va suivre, qui se réfère à des exemples de mise en oeuvre de l'invention ainsi qu'aux dessins annexés, dans lesquels :
- La Figure 1 représente les structures chimiques d'un noyau dicétopipérazine (A) et de l'albonoursine (B).
- La Figure 2 représente un schéma de la région génomique de *Streptomyces noursei* incluant le cluster de gènes nécessaire à la synthèse de l'albonoursine, dans laquelle orf1 correspond à albA, orf 2 correspond à albB, orf3 correspond à albC et orf 4 correspond à albD.
- La Figure 3 représente la voie de synthèse supposée de l'albonoursine dans *Streptomyces noursei.*
- La Figure 4 représente certaines constructions plasmidiques réalisées pour l'introduction des différentes phases ouvertes de lecture (*open reading frame* ou orf) dans *Escherichia coli* ou *Streptomyces lividans*.
- La Figure 5 représente les résultats des analyses des milieux de culture de *Streptomyces lividans*, transformé par introduction des plasmides pSL128 (A), pUWL201 (B) et pSL129 (C).
- La Figure 6 représente les résultats des analyses des milieux de culture de *Streptomyces lividans*, transformé par introduction des plasmides pSL168 et pSL159, ou de *Streptomyces lividans* non transformé :
   A : *S*. *lividans*[pSL168], incubé en présence de CDO ;
   B : *S*. *lividans*[pSL168], incubé en absence de CDO ;
   C : *S. lividans[pSL159]* en absence ou en présence de CDO ;
   D : *Streptomyces lividans* TK21 incubé en présence de CDO.
- La Figure 7 représente l'analyse par gel de polyacrylamide/sodium dodécylsulfate (SDS-PAGE) de l'expression de AlbA et AlbB₁ et AlbB₂ à partir du polynucléotide albA-albB dans *E*. *coli* BL21(DE3)-plysS. Coloration au bleu de Coomassie brillant R-250. Ligne S : Standard de poids moléculaire, ligne 1 : extrait cytoplasmique total (environ 10 µg), ligne 2 : fraction enzymatique purifiée sur colonne Ni-sépharose (environ 10 µg).

Les exemples suivants sont illustratifs de l'invention et ne la limitent aucunement.

### EXEMPLE 1 : Isolement du polynucléotide de l'invention chez Streptomyces noursei.

Un polynucléotide contenant l'ensemble de l'information génétique nécessaire pour la biosynthèse de l'albonoursine chez *Streptomyces noursei* a été isolé du génome total de ce microorganisme, par une approche basée sur l'amplification génique par PCR.

### - Obtention de séquences peptidiques partielles de la cyclodipeptide oxydase :

Des informations partielles de séquences peptidiques sur l'enzyme catalysant, chez *Streptomyces noursei*, la conversion du cyclodipeptide cyclo(L-Phe-L-Leu) en albonoursine, nommée cyclodipeptide oxydase (CDO), ont été obtenues par séquençage direct par la méthode d'Edman de polypeptides issus de l'hydrolyse trypsique de l'enzyme purifiée selon le protocole décrit dans Gondry M. et al. (Gondry et al. 2001, précité). Après séparation des constituants de la fraction enzymatique par électrophorèse en gel de polyacrylamide à 15 % et coloration des protéines au bleu de Coomassie, une bande de gel contenant la protéine de masse moléculaire d'environ 21.000 daltons est découpée et incubée dans 1 ml de tampon Tris-HCl 50 mM pH 8, en présence de trypsine (concentrations relatives trypsine/substrat = 1/50), pendant 20 heures à 37°C. Les polypeptides obtenus sont ensuite séparés par chromatographie liquide haute performance (HPLC) en phase inverse (colonne µRPC C₂/C₁₈ SC21/10, Pharmacia) par un gradient linéaire de 0 % à 76 % en acétonitrile en 62 minutes (solvant : 0,1 % acide trifluoroacétique; débit : 1ml/min). Chacun des polypeptides séparés est ensuite purifié par chromatographie de perméation de gel sur une colonne superdex peptide PC32/30 (Pharmacia) équilibrée en tampon contenant 30 % d'acétonitrile et 0,1 % d'acide trifluoroacétique. Trois des polypeptides obtenus ont été finalement analysés par séquençage automatique par la méthode d'Edman (séquenceur modèle 477A, Applied Biosystems) et par spectrométrie de masse MALDI-TOF. Le Tableau I présente les séquences peptidiques obtenues ainsi que les séquences nucléotidiques qui en ont été déduites.

**Tableau I :**

| **Séquence peptidique** | **Séquence nucléotidique déduite** |
|---|---|
| **EPVDDA**LIEQLLEAMLAAPT (SEQ ID N°11) | GARCCSGTSGACGACGC (oligo1f) (SEQ ID N°14) |
| | GCGTCGTCSACSGGYTC (oligo1r) (SEQ ID N°15) |
| **NEVVNY**EXWGNR (SEQ ID N°12) | AACGARGTSGTSAACTACGA (oligo2f) (SEQ ID N°16) |
| | TCGTAGTTSACSACYTCGTT (oligo2r) (SEQ ID N°17) |
| **QAXSFMV**VR (SEQ ID N°13) | CAGGCSTGGWSSTTCATGGT (oligo3f) (SEQ ID N°18) |
| | ACCATGAASSWCCASGCCTG (oligo3r) (SEQ ID N°19) |

| | |
|---|---|
| X : acide aminé indéterminé. (R = A or G; S = C or G; Y = C or T and W = A or T). | |

La comparaison des masses expérimentale et théorique du polypeptide correspondant à la séquence SEQ ID N°13 permet l'identification d'un résidu tryptophane en position 3. Les séquences soulignées et en gras ont été utilisées pour concevoir 6 oligonucléotides dégénérés, sens (1f, 2f et 3f) et antisens (1r, 2r et 3r), en fonction de l'utilisation typique des codons chez *Streptomyces*. En l'absence d'informations sur la position respective des polypeptides dans la séquence protéique, une combinaison des six oligonucléotides a été utilisée pour le clonage. Les conditions de PCR testées conduisant à un nombre de fragments nucléotidiques amplifiés trop important, une stratégie de transcription inverse (RT-PCR) a été développée.

### - Amplification d'un fragment oligonucléotidique par RT-PCR :

L'ARN total de *Streptomyces noursei* a été extrait à partir d'une culture de 24 heures en milieu 5 (milieu ATCC) selon le protocole décrit par Kieser et al. (Practical Streptomyces Genetics. The John Innes Foundation Norwich, U.K. (2000)). Un traitement supplémentaire à la DNase I permet d'éliminer complètement l'ADN. Les oligonucléotides dégénérés (sens et antisens) ont été synthétisés par Sigma Genosys Ltd et la RT-PCR a été réalisée en utilisant le kit Titan™ One Tube RT-PCR (Boehringer Mannheim) selon les instructions standard, avec 1 µg d'ARN total pour chaque réaction. La transcription inverse est réalisée à 50°C pendant 30 minutes, puis les conditions de PCR sont les suivantes : dénaturation initiale à 97°C pendant 4 min, suivie de 45 cycles de 1 min à 95°C, 1 min à 50°C et 1 min à 68°C, et la réaction finale de polymérisation à 68°C pendant 10 minutes. Les produits de réaction sont analysés par électrophorèse en gel d'agarose 1,5 %, puis purifiés (DNA and Gel Band purification kit, Pharmacia).

La RT-PCR avec les six combinaisons d'oligonucléotides a conduit à l'amplification d'un fragment unique d'environ 400 paires de bases avec les oligonucléotides 3f et 2r. Ce fragment a été cloné dans le vecteur pGEM-T easy vector et séquencé, permettant de confirmer que les amorces oligonucléotidiques utilisées sont bien dans le même cadre de lecture. Ce fragment nucléotidique a été utilisé comme sonde pour cribler une librairie d'ADN génomique de *Streptomyces noursei*, préparée dans le cosmide pWED1.

### - Construction d'une librairie d'ADN génomique de Streptomyces noursei :

L'ADN génomique (2,5 µg), extrait de *Streptomyces noursei* selon les procédures standard (Kieser et al., précité ; Sambrook J. et al, Molecular cloning: a laboratory manual. Cold Spring Harbor Laboratory Press. Cold Spring Harbor, N.Y. (1989)) a été partiellement digéré avec 0,33 U de BamHI, conduisant à des fragments d'ADN d'environ 35 à 45 kb. Ces fragments sont introduits par ligation dans le vecteur cosmidique pWED1 préalablement digéré par BamHI et déphosphorylé. Le produit de ligation a été encapsidé *in vitro* dans des phages lambda (Packagene Lambda DNA packaging system, Promega) et introduit par transfection dans la souche *E*. *coli* (SURE).

### - Criblage de la librairie d'ADN génomique de Streptomyces noursei :

Le fragment nucléotidique amplifié par RT-PCR a été ensuite marqué par amorçage aléatoire avec du [α-³²P]-dCTP en utilisant le kit T7 Quick Prime (Pharmacia) et utilisé comme sonde pour cribler la banque. Environ 2000 clones ont été testés par hybridation sur colonies selon la méthode standard (Sambrook J et al., précité) et 12 clones ont été sélectionnés. Les cosmides correspondants (notés pSL110 à pSL121) ont été extraits, digérés par BamHI et analysés par la technique de Southern blot en utilisant le fragment de RT-PCR comme sonde. Cette sonde a permis d'isoler un fragment nucléotidique de 3,8kb, commun à tous les cosmides et également présent dans l'ADN génomique de *Streptomyces noursei* digéré par BamHI. Ce fragment BamHI a été isolé à partir du cosmide pSL117 et cloné dans le vecteur pBC SK+, pour donner le vecteur pSL122 (Définition des vecteurs utilisés : cf. Tableau II).

### EXEMPLE 2 : Analyse de la séquence du polynucléotide de l'invention.

Le séquençage automatique des polynucléotides de l'invention a été réalisé sur un analyseur ABI PRISM Genetic Analyzer (Perkin Elmer) en utilisant le kit DYEnamic ET terminator cycles (Pharmacia) ou par la société Genome Express. L'analyse informatique des séquences et les comparaisons avec les banques de données ont été réalisées avec les programmes Frame (Bibb, M.J. et al., Gene, 30, 157-166 (1984)), BLAST et FASTA (Altschul,S. F. et al., Nucleic Acids Res., 25, 3389-3402 (1997); Pearson, W. R., Methods in Enzymology, 183, 63-98 (1990).

L'analyse par le programme FRAME du polynucléotide BamHI (SEQ ID N°5), fait apparaître quatre phases de lecture ouvertes complètes, notées orf1 à orf4, (albA à albD, SEQ ID N°1 à 4) transcrites dans la même direction, et une phase ouverte de lecture (1119 bp) dont l'extrémité est tronquée, notée orf5 (voir Figure 2). Le produit de traduction de orf5 présente un très fort degré de similitude avec la partie N-terminale d'une glutamate déshydrogenase NADP-spécifique de *Streptomyces coelicolor* (78 % d'identité et 86 % de similitude selon le programme BLAST).

La première des phases ouvertes de lecture, orf1 (albA, SEQ ID N°1), contient la séquence nucléotidique du fragment amplifié par RT-PCR, et la séquence peptidique déduite contient bien la séquence des 3 peptides trypsiques initialement isolés. Le produit de orf1 correspond par conséquent à la protéine enzymatique de masse d'environ 21 kDa isolée et purifiée de *Streptomyces noursei* (Gondry M. et al.; précité). Par conséquent ce gène est bien impliqué dans la biosynthèse de l'albonoursine et sera désigné par albA.

L'analyse de la séquence de albA (orf1) indique que 3 codons, deux GUG et un AUG, pourraient être considérés comme codons d'initiation pour la traduction d'albA, ce qui aboutirait à des protéines de 219, 204 ou 196 aminoacides. Des tentatives de détermination de la séquence peptidique N-terminale ayant échoué, du fait de la présence d'une modification post-traductionnelle sur cette extrémité, la séquence la plus longue (657 nucléotides) a été retenue pour albA (SEQ ID N°6). (Le premier codon d'initiation est situé à 20 nucléotides de distance de l'extrémité du fragment BamHI qui, par conséquent, ne contient pas la région promotrice qui doit se situer plus en amont de ce gène).

La comparaison de la séquence peptidique déduite de alba (AlbA, SEQ ID N°6) avec les bases de données a montré un degré de similitude maximal avec une NADH oxydase de *Archaeoglobus fulgidus* (32% d'identité et 52 % de similitude selon le programme BLAST) et la recherche de domaines conservés indique qu'il présente un large domaine de type nitro-réductase (pfam00881, 151 aminoacides).

Contigu à albA, mais en décalage de cadre de lecture, orf2 (albB, SEQ ID N°2) présente également l'utilisation typique des codons de Streptomyces. albB est traduit sous deux isoformes (AlbB₁, SEQ ID N°7 et AlbB₂, SEQ ID N°8) nécessaires à l'activité du polypeptide AlbA, selon le codon d'initiation, AUG ou GUG, pris en considération pour orf2. Les deux isoformes d'AlbB qui sont exprimées en quantité à peu près équivalentes, se différencient par la présence de 5 acides aminés supplémentaires localisés à l'extrémité N-terminale d'AlbB₁ et résultant de l'utilisation de deux codons d'initiation différents. Dans le cas d'AlbB₁, la méthionine initiale est éliminée.

Les 2 possibilités sont compatibles avec l'analyse de la séquence avec le programme FRAME. Les programmes BLAST et FASTA ne révèlent aucune homologie particulière entre la séquence peptidique déduite de orf2 et les protéines des banques de données.

De la même façon, les recherches dans les banques de données réalisées à partir des séquences polypeptidiques, déduites respectivement de orf3 (albC, SEQ ID N°3) et orf4 (albD, SEQ ID N°4) ne révèlent aucune homologie significative avec une protéine de fonction connue. orf3 débute par un codon d'initiation ATG et code pour un polypeptide de 239 acides aminés, AlbC (SEQ ID N°9) qui présente un faible degré de similitude avec deux protéines hypothétiques de fonction inconnue : Rv2275 de *Mycobacterium tuberculosis* (34 % d'identité et 53 % de similitude selon le programme BLAST) et YvmC de *Bacillus subtilis* (29 % d'identité et 46 % de similitude selon BLAST). Orf4 code pour une protéine de 277 acides aminés, AlbD (SEQ ID N°10) qui comprend un domaine membranaire, comme l'indique l'analyse de sa séquence avec le programme TMHMM (Krogh, A. et al., J. Mol. Biol. 305, (2001), et présente une faible homologie avec une protéine membranaire de fonction inconnue de *Streptomyces coelicolor* (54 % d'identité et 67 % de similitude selon BLAST).

### EXEMPLE 3 : Clonage des polynucléotides albA, albB, albC et albD et construction des vecteurs d'expression.

Les procédés d'extraction et de préparation d'ADN, de transformation des souches *Escherichia coli* et *Streptomyces lividans* TK 21, de préparation des protoplastes ont été réalisés selon les protocoles standard décrits par Sambrook et al. (précité) et Kieser et al. (précité).

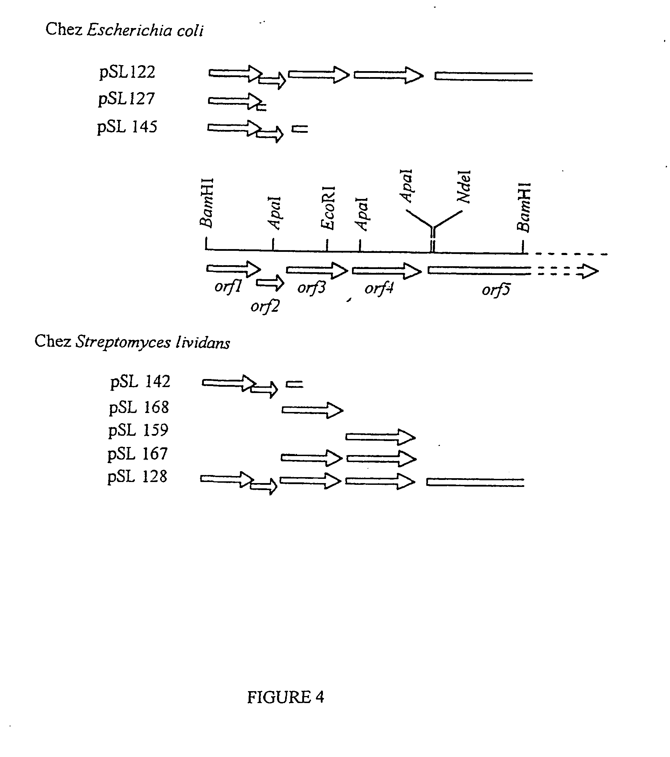

L'ensemble des vecteurs plasmidiques et cosmidiques préparés pour manipuler les polynucléotides faisant l'objet de la présente invention sont présentés dans le Tableau II (voir aussi Figure 4).

**Tableau II : Souches et vecteurs utilisés**

| **Bactéries** | **Propriétés** | **Source/référence** |
|---|---|---|
| *E. coli* DH5α | souche standard pour le clonage | Invitrogen |
| *E. coli* SURE | souche utilisée pour les librairies de cosmides | Stratagene |
| *Streptomyces lividans* TK21 | souche *Streptomyces* pour le clonage des gènes alb | Hopwood, D. A. et al. J. Gen. Microbol. 129, 2257-2269 (1983). |
| *Streptomyces noursei* ATCC 1145 | souche sauvage produisant l'albonoursine | ATCC |

| **Vecteurs** | | |
|---|---|---|
| pGEM-T easy | Vecteur de clonage des produits de PCR, Amp^{R} | Promega |
| pWED1 | Vecteur cosmidique dérivé de pWE15, Amp^{R} | Gourmelen, A. et al., Antimicrobial Agents Chemotherapy, 42, 2612-2619 (1998) |
| pBC SK⁺ | Vecteur de clonage, Cm^{R} | Stratagene |
| pHP45 Ωaac | Plasmide utilisé comme source pour la cassette Ωaac, Amp^{R}, Apr^{R} | Blondelet-Rouault, M. H. et al., Gene, 190, 315-317 (1997). |
| pUWL201 | Vecteur navette *E. coli*/*Streptomyces,* contient le promoteur ErmE* pour l'expression des gènes clonés dans *Streptomyces.* Amp^{R}, Thio^{R} | Doumith, M., et al., Mol. Gen. Genet. 264, 477-485 (2000) |
| pET-28a | Vecteur d'expression | Novagen |
| pSL117 | Cosmide de la librairie de *Streptomyces noursei*, utilisé pour les produits de RT-PCR | |
| pSL122 | Fragment BamHI de 3.8 kb, cloné dans pBC SK⁺ | |
| pSL127 | Dérivé de pSL122, comprenant une délétion interne du fragment Apal, supprimant orf2, orf3, orf4 et orf5. | |
| pSL128 | Fragment BamHI de 3.8 kb de pSL122, cloné dans pUWL201, sous le contrôle de ErmE*p. | |
| pSL129 | Fragment BamHI de 3.8 kb de pSL122, cloné dans pUWL201, contenant l'insertion dans la direction opposée à pSL128. | |
| pSL138 | Dérivé de pSL122, comprenant une délétion interne du fragment EcoRI, supprimant orf3, orf4 et orf5, et avec insertion de la cassette Ωaac. | |
| pSL140 | Dérivé de pSL122, comprenant une délétion interne du fragment NdeI/EcoRV supprimant orf5, et avec insertion de la cassette Ωaac. | |
| pSL142 | Fragment Asp718/Klenow/BamHI de pSL138 (contenant orf1, orf2, et la cassette Ωaac) cloné dans pUWL201. | |
| pSL144 | Fragment Asp718/Klenow/BamHI de pSL140 (contenant orf1 à orf4 et la cassette Ωaac) cloné dans pUWL201. | |
| pSL145 | Dérivé de pSL122 avec délétion interne du fragment EcoRI, supprimant orf3, orf4 et orf5 | |
| pSL150 | Produit de PCR de l'amplification de orf1-orf2, cloné dans le vecteur d'expression pET-28a. | |
| pSL157 | Produit de PCR de l'amplification de orf4, cloné dans pGEM-T easy. | |
| pSL159 | Fragment Pstl/Klenow/BamHI de pSL157 cloné dans pUWL201. | |
| pSL165 | Produit de PCR de l'amplification de orf3 cloné dans pGEM-T easy. | |
| pSL166 | Produit de PCR de l'amplification de orf3 + orf4 cloné dans pGEM-T easy. | |
| pSL167 | Fragment Pstl/Klenow/BamHI de pSL166 cloné dans pUWL201 | |
| pSL168 | Fragment PstIl/Klenow/BamHI de pSL165 cloné dans pUWL201 | |

| | | |
|---|---|---|
| pSL117 est un cosmide contenant la librairie d'ADN génomique de *Streptomyces noursei.* pSL122 contient le polynucléotide BamHI (SEQ ID N°5) de 3,8 kb de *Streptomyces noursei* faisant l'objet de l'invention, cloné dans le vecteur de clonage pBC SK⁺. pSL127 et pSL145 ont été construits respectivement par digestion de pSL122 par Apal ou EcoRI, et religation. | | |

Le polynucléotide BamHI a également été cloné dans le vecteur navette *E. coli*/*streptomyces* pUWL201, dans l'orientation adéquate pour avoir tous les gènes sous le contrôle du promoteur ermE* (pSL128) ou bien dans l'orientation opposée (pSL129).

pSL142 et pSL144 ont été construits en 2 étapes : pSL122 a d'abord été digéré par EcoRI et l'enzyme de Klenow ou Ndel et l'enzyme de Klenow, puis une ligation entre ces fragments et la cassette Ωaac digérée par Hindlll-Klenow conduit aux plasmides pSL138 et pSL140. Ces plasmides sont ensuite digérés par Asp718, Klenow et BamHI, et les fragments obtenus contenant orf1 (albA, SEQ ID N°1), orf2 (albB, SEQ ID N°2) et la cassette Ωaac, pour le premier, et orf1 à orf4 (albA à albD) et la cassette Ωaac, pour le second, sont clonés dans le vecteur pUWL201 digéré par Xbal-Klenow-BamHI.

orf3 (albC, SEQ ID N°3), orf4 (albD, SEQ ID N°4) et (orf3+orf4) sont amplifiées par PCR en utilisant les amorces suivantes :
pour orf3
   sylv24 : (SEQ ID N°20) :
   5'-CGGCTGCAGGAGAAGGGAGCGGACATATGCTTGCAGGCTTAGTTCCC -3', (site PstI souligné) ;
   sylv22 : (SEQ ID N°21) :
   5'-CGGTCCCGTGGATCCAAGCTTCTAGGCCGCGTCGGCCAGCTC-3', (site BamHI souligné) ;
pour orf4
   sylv19 : (SEQ ID N°22) :
   5'-GAGCGGGATCCTGCAGTGTCATGGGGAGGACAGGAC-3', (site Pstl souligné) ; sylv18 : (SEQ ID N°23) :
   5'-CGATCACGTGGATCCAAGCTTGCCAATCCTGTACGCGATTT-3', (site BamHI souligné) ;
pour (orf3+orf4) : sylv24 et sylv18.

orf2 et orf3 étant séparés seulement de 37 nucléotides, un site synthétique de liaison au ribosome a été inclus dans sylv24 afin d'assurer une bonne traduction de orf3. Les fragments amplifiés par PCR ont ensuite été clonés dans le vecteur pGEM-T easy (Promega) pour donner pSL165, pSL157 et pSL166. Les fragments PstI-BamHI obtenus à partir de ces trois plasmides ont ensuite été clonés dans le vecteur pUWL201 digéré par Pstl-BamHI pour donner pSL168, pSL159 et pSL167.

### EXEMPLE 4: Production du dérivé de dicétopipérazine cyclo(ΔPhe-ΔLeu) (albonoursine) chez un hôte hétérologue, Streptomyces lividans, transformé par introduction du polynucléotide BamH1 (SEQ ID N°5).

Des protoplastes de *Streptomyces lividans* TK21 ont été transformés par pSL128 (contenant le polynucléotide BamH1 (SEQ ID N°5)), pSL129 ou pUWL201 (contrôle) selon les protocoles standard décrits par Sambrook et al. (précité) et Kieser et al. (précité). La culture des trois souches a été réalisée en milieu M5 (milieu ATCC), milieu riche contenant un large excès d'acides aminés, pendant 3 jours à une température comprise entre 28 et 30°C. Les surnageants des cultures des 3 souches transformées ont été analysés par HPLC en phase inverse dans les conditions suivantes : le surnageant des cultures (500 µl) a été filtré (ultrafree-MC 10 kDa, Millipore) et injecté directement en HPLC (colonne C18 (4.6 x 250 mm) Vydac : debit : 1 ml/min ; élution : gradient linéaire de 0 à 45 % d'acétonitrile dans 0,1 % d'acide trifluoroacétique en 45 minutes. L'élution a été suivie à l'aide d'un détecteur multi-longueur d'onde entre 200 et 600 nm.

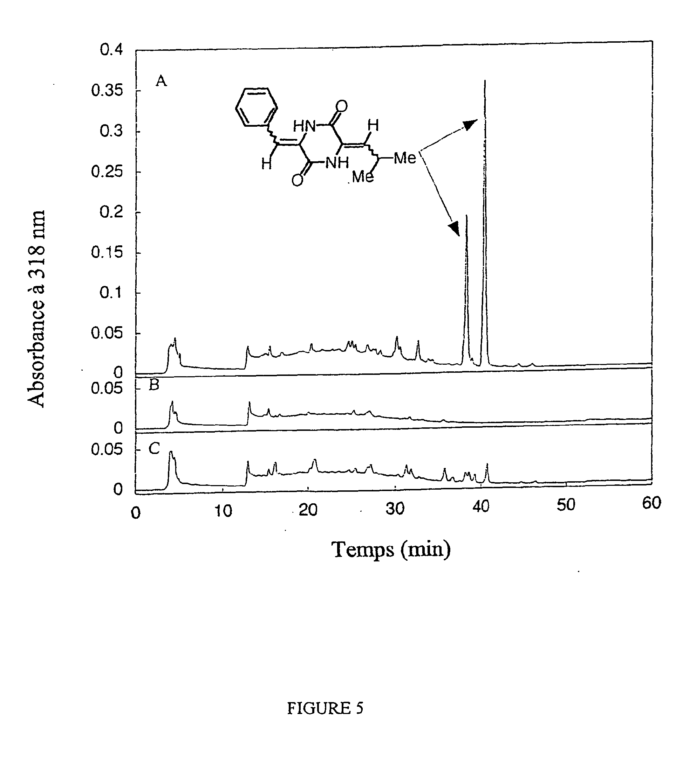

On détecte chez *S. lividans*[pSL128] la production d'albonoursine sous 2 formes stéréoisomériques (2 pics à 38,3 min et 40,5 min ; λₘₐₓ = 318 nm ; m = 256,4 Da). (Figure 5A)

Dans les souches contrôles *S. lividans*[pUW201] et *S. lividans*[pSL129] (Figure 5B et 5C) on ne détecte aucune production d'albonoursine.

Le polynucléotide BamHI (SEQ ID N°5) de *Streptomyces noursei* contient l'ensemble de l'information génétique pour la production d'albonoursine.

### EXEMPLE 5 : Mise en évidence de la fonction des polynucléotides albA et albB par visualisation de la conversion de cyclo(L-Trp-L-Trp) en cyclo(ΔTrp-ΔTrp) sur boîte de Pétri à partir d'un hôte hétérologue, Escherichia coli, transformé par insertion des polynucléotides albA et albB.

Un test rapide sur boîte de Pétri a été mis au point pour détecter directement la conversion de cyclodipeptides en bis-déshydro-cyclodipeptides sur colonies isolées. Ce test repose sur la conversion du cyclodipeptide, incolore en solution cyclo(L-Trp-L-Trp), en un produit jaune et insoluble cyclo(ΔTrp-ΔTrp) (λₘₐₓ = 367 nm et 450 nm), conférant une couleur jaune vif aux colonies présentant l'activité CDO.

Les souches *E. coli*, transformées avec les plasmides préparés selon le protocole détaillé à l'exemple 3, ont été testées directement sur boîte de milieu LB contenant 0,5 mM de cyclo(L-Trp-L-Trp).

Après 16 heures d'incubation à 37°C, les souches *E. coli*[pSL122] (contenant le polynucléotide BamH1) et *E. coli*[pSL145] (contenant le fragment BamH1 avec une délétion de orf3 à orf5) présentent une coloration jaune intense, alors que *E. coli*[pBC SK+] (contenant le vecteur de clonage intact) et *E. coli*[pSL127] (contenant le fragment BamH1 avec une délétion de orf2 à orf5) ne sont pas colorées.

Ce résultat démontre l'implication des deux gènes orf1 (albA, SEQ ID N°1) et orf2 (albB, SEQ ID N°2), dans l'activité cyclodipeptide oxydase associée à la production d'albonoursine.

### EXEMPLE 6 : Expression de l'activité enzymatique cyclodipeptide oxydase (CDO) chez un hôte hétérologue, Streptomyces lividans, transformé par introduction de orf1 et orf2 (albA et albB, SEQ ID N°1 et SEQ ID N°2).

L'analyse par HPLC du surnageant de culture de la souche *Streptomyces lividans* TK21, transformée par le plasmide pSL142 contenant le polynucléotide BamH1 délété de orf3 (albC, SEQ ID N°3), orf4 (albD, SEQ ID N°4) et orf5, dans les conditions de culture décrites à l'exemple 4, démontre l'absence de production d'albonoursine, et indique donc l'implication de orf3 et/ou orf4 dans la production du dérivé de dicétopipérazine.

Pourtant, l'addition dans le surnageant de cyclo(L-Phe-L-Leu), dans les conditions standard décrites dans Gondry et al. (précité), aboutit bien à la production d'albonoursine. Ceci suggère l'implication de orf3 et/ou orf4 dans la biosynthèse du cyclodipeptide cyclo(L-Phe-L-Leu).

### EXEMPLE 7 : Mise en évidence de la production de cyclo(L-Phe-L-Leu) et cyclo(L-Phe-L-Phe) in vivo chez un hôte hétérologue, Streptomyces lividans, transformé par introduction de orf3 (albC, SEQ ID N°3).

Pour confirmer les résultats décrits dans l'exemple 6, orf3 (albC) et orf4 (albD) ont été clonés séparément dans le plasmide navette pUWL201, donnant respectivement pSL168 (orf3) et pSL159 (orf4), qui ont été introduits dans *Streptomyces lividans* TK21 selon les protocoles standard décrits par Sambrook et al. (précité) et Kieser et al. (précité).

Après culture dans les conditions décrites à l'exemple 4, les surnageants de culture de *S. lividans*[pSL168] et *S. lividans*[pSL159] ont été analysés par HPLC, dans les conditions standard décrites dans l'exemple 4, afin de mettre en évidence la production du cyclodipeptide cyclo(L-Phe-L-Leu). La détection directe de ce composé étant difficile du fait de son faible coefficient d'absorption molaire (εₘₒₗ ≈ 200 M⁻¹cm⁻¹ à 254 nm) et de la complexité du milieu, sa mise en évidence a été réalisée après conversion du cyclodipeptide en albonoursine (cyclo(ΔPhe-ΔLeu), εₘₒₗ = 25120 M⁻¹.cm⁻¹ à 318 nm), par addition de l'enzyme CDO purifiée selon le procédé décrit dans Gondry et al. (précité).

Les surnageants de culture ont été filtrés, puis incubés pendant 10 à 15 heures à 30°C avec 4.1x10⁻³ unités enzymatiques de CDO purifiée. L'analyse HPLC comparée des surnageants de culture, incubés ou non avec CDO, a été réalisée La masse moléculaire des métabolites produits a été déterminée par spectrométrie de masse (Quattro II, Micromass).

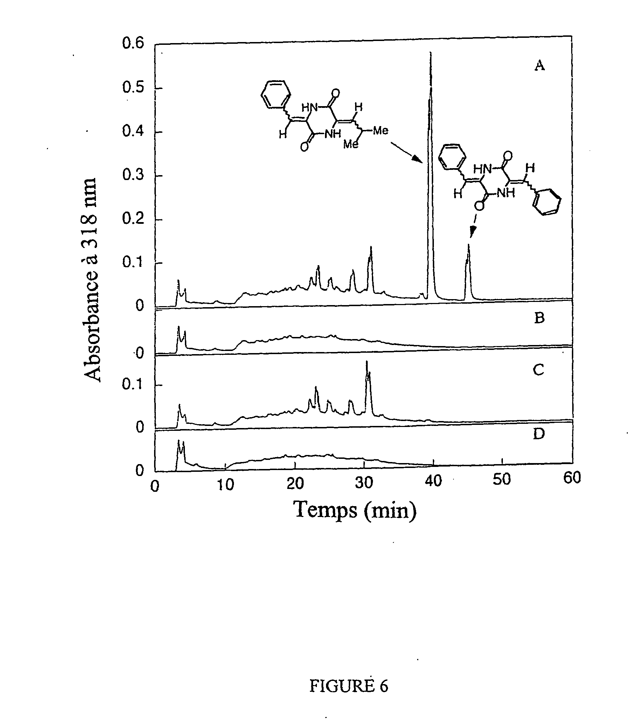

Les résultats sont présentés sur la Figure 6 :
On détecte dans le surnageant de culture de S. lividans[pSL168], incubée en présence de CDO, de l'albonoursine (cyclo(ΔPhe-ΔLeu)) (pic à 40,5 min ; λₘₐₓ = 318 nm ; m = 256,4 Da) et du cydo(ΔPhe-ΔPhe) (pic à 44,1 min ; λₘₐₓ = 338 nm ; m = 290,3) (panneau A) ; Aucun métabolite n'est détecté :
   - dans le surnageant de culture de *S. lividans*[pSL168] en absence de CDO (panneau B) ;
   - dans le surnageant de culture de *S. lividans*[pSL159] en absence ou en présence de CDO (panneau C) ;
   - dans le surnageant de culture de *Streptomyces lividans* TK21 incubée en présence de CDO : (panneau D).

Ce résultat démontre clairement l'implication de orf3 (albC) dans la production des cyclodipeptides cyclo(L-Phe-L-Leu), précurseur de l'albonoursine, et cyclo(L-Phe-L-Phe), précurseur d'un second métabolite, cydo(ΔPhe-ΔPhe), produit conjointement au premier chez *Streptomyces noursei* (Khokhlov A.S. et al., Tetrahedron Lett., 27, 1881 (1963)).

### EXEMPLE 8 : Mise en évidence de la production de cyclo(L-Phe-L-Leu) et cyclo(L-Phe-L-Phe) in vivo chez un hôte hétérologue, Streptomyces lividans, transformé par introduction du polynucléotide orf3-orf4 (albC-albD, SEQ ID N°3-SEQ ID N°4).

Selon une variante de l'exemple 7, le polynucléotide orf3-orf4 (albC-albD) a été cloné dans le plasmide navette pUWL201 et le plasmide résultant, pSL167, a été introduit dans *Streptomyces lividans* TK21 selon les protocoles standard décrits par Sambrook et al. (précité) et Kieser et al. (précité).

L'analyse HPLC du surnageant de culture, traité de façon identique au procédé décrit dans l'exemple 7, montre que l'on détecte dans le surnageant de culture de *S. lividans*[pSL167] incubée en présence de CDO de l'albonoursine (cyclo(ΔPhe-ΔLeu)) et du cyclo(ΔPhe-ΔPhe) et qu'aucun métabolite n'est détecté dans le surnageant de culture de *S. lividans*[pSL167] en absence de CDO, ni dans le surnageant de culture de *Streptomyces lividans* TK21 incubée en présence de CDO.

Ces résultats démontrent que le produit du gène orf5 ne participe pas directement à la biosynthèse de l'albonoursine, tandis que orf4, (albC), est nécessaire et suffisante pour produire les cyclodipeptides précurseurs cyclo(L-Phe-L-Leu) et cyclo(L-Phe-L-Phe), chez *Streptomyces lividans* comme chez *Streptomyces noursei.*

### EXEMPLE 9 : surexpression de AlbA et AlbB à partir du polynucléotide albA-albB (SEQ ID N°1-SEQ ID N°2).

Le vecteur pET-28a(+), qui contient une séquence N-terminale ou C-terminale poly-histidine (His-tag), a été utilisé pour la construction d'un vecteur d'expression contenant le polynucléotide albA-albB, afin de faciliter la purification de la protéine recombinante. La séquence la plus courte de albA, codant pour un polypeptide de 196 résidus d'aminoacides, a été choisie. L'amplification génique du polynucléotide par PCR a été réalisée en utilisant des amorces oligonucléotidiques conçues pour inclure un site de clonage Ndel (sens) et Xhol (antisens).

Les conditions de PCR sont les suivantes : dénaturation initiale à 94°C pendant 4 min suivie de 10 cycles d'1 minute à 94°C, 1 minute à 45°C et 1,5 min à 72°C, puis par 20 cycles d'1 minute à 94°C, 1 minute à 50°C et 1,5 min à 72°C, et une réaction de polymérisation finale à 72°C pendant 10 min. Les produits de réaction ont été digérés par Ndel et Xhol, et les fragments sous-clonés dans le vecteur pET-28a pour donner pSL150. La séquence de l'insert a été contrôlée par séquençage automatique (ABI PRISM Genetic analyzer, Perkin Elmer) en utilisant le kit DYEnamic ET terminator cycles (Amersham Pharmacia Biotech).

Des conditions standard d'expression ont été utilisées :
- température de croissance : 20°C,
- induction de l'expression : 0,6 mM IPTG
- durée de l'induction : 16 heures.

pSL150 a été introduit dans *E. coli* BL21(DE3)-plysS. La souche a été cultivée en milieu LB à 20°C jusqu'à une absorbance de 0.6, puis l'expression est induite. La culture est alors centrifugée à 4190 g, à 4°C pendant 15 min. Les cellules ont été remise en suspension en tampon d'extraction (100 mM Tris-HCl pH 8,0, 1 µM phosphoramidon, 1 mM PMSF et 5 % glycérol), et broyées avec une presse d'Eaton. L'extrait protéique est incubé en présence de benzonase (25 U/ml) à 30°C pendant 10 min, puis centrifugé à 11300 g pendant 15 min à 4°C. L'activité enzymatique a été déterminée selon le test standard décrit par Gondry et al. (précité). Une unité d'activité enzymatique est définie comme la quantité d'enzyme catalysant la production d'1 µmole de produit par minute, et l'activité spécifique est exprimée en unités d'enzyme par mg de protéines. L'activité spécifique de l'extrait enzymatique est augmentée d'un facteur 50 (As = 2 U/mg) après purification par chromatographie d'affinité (colonne : HiTrap chelating HP (1 ml), Amersham Pharmacia Biotech ; équilibration en ions Ni²⁺ en tampon 100 mM Tris-HCl pH 8.0, 0,5 M NaCl et 10 mM imidazole ; élution : gradient de 0,3 à 1 M imidazole en 35 min ; débit : 1 ml/min).

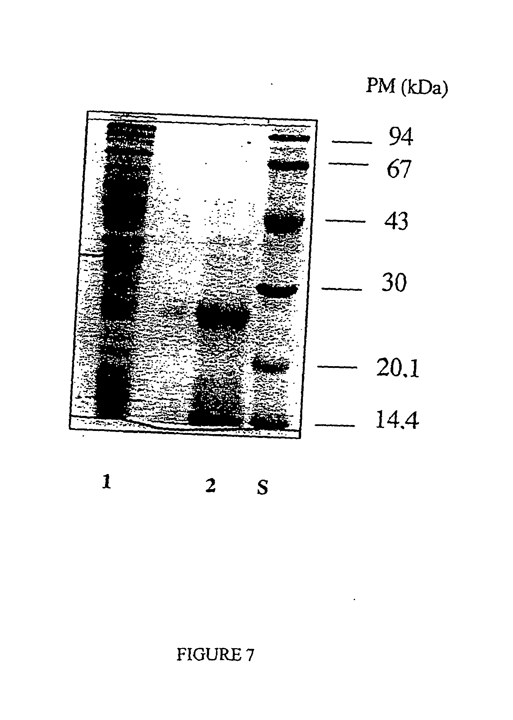

L'analyse par gel de polyacrylamide / sodium dodécylsulfate (SDS-PAGE) (12 %) de la fraction purifiée de *E. coli*[pSL150] (Figure 7) démontre la présence simultanée de AlbA et AlbB en proportions non stoechiométriques, et démontre que AlbB est exprimée sous 2 isoformes (2 bandes en SDS-PAGE). L'analyse de AlbB purifiée par spectrométrie de masse et séquençage N-terminal de la séquence polypeptidique indique que ces deux formes correspondent aux produits AlbB₁ et AlbB₂ à partir des 2 codons d'initiation identifiés dans sa séquence (cf. ci-dessus).

### EXEMPLE 10 : Conversion in vitro de cyclo(L-Phe-L-His) et cyclo(L-Phe-L-Leu) par AlbA-AlbB recombinant.

La préparation enzymatique purifiée selon le procédé décrit dans l'exemple 9, incubée dans les conditions standard telles que décrites par Gondry et al. (précité), catalyse la conversion *in vitro* de cyclodipeptides en monodéshydro- et bisdéshydro-cyclodipeptides.

La préparation enzymatique purifiée a été incubée en présence des substrats cyclo(L-Phe-L-His) et pendant 72 h à 30°C. Les produits de la réaction ont été analysés par HPLC en phase inverse dans les conditions décrites dans l'exemple 4, et identifiés sur la base de leurs caractéristiques spectrales et de leur masse moléculaire confirmée par spectrométrie de masse. Les produits de la réaction sont les suivants :
- à partir de cyclo(L-Phe-L-His) : cyclo(ΔPhe-L-His)at (λₘₐₓ = 297 nm et m = 282 Da) et cyclo(ΔPhe-ΔHis) (λₘₐₓ = 338 nm et m = 280 Da)
- à partir de cyclo(L-Phe-L-Leu) : cyclo(ΔPhe-L-Leu) (λₘₐₓ = 297 nm et m = 258 Da) et cyclo(ΔPhe-ΔLeu) (λₘₐₓ = 316 nm et m = 256 Da)

Ces résultats confirment que la préparation enzymatique obtenue à partir du clonage du polynucléotide albA-albB dans un vecteur d'expression introduit chez un hôte hétérologue catalyse *in vitro* la conversion de cyclodipeptides en dérivés α,β-déshydrogénés des dicétopipérazines.

## Revendications

1. Polynucléotide isolé ou synthétique, **caractérisé en ce qu'**il comprend au moins les trois phases ouvertes de lecture correspondant aux séquences SEQ ID N°1, SEQ ID N°2 et SEQ ID N°3.

2. Polynucléotide selon la revendication 1, **caractérisé en ce qu'**il comprend en outre la phase ouverte de lecture correspondant à la séquence SEQ ID N°4.

3. Polynucléotide isolé, naturel ou synthétique, **caractérisé en ce qu'**il répond à la séquence SEQ ID N°5.

4. Polynucléotide isolé, naturel ou synthétique, **caractérisé en ce qu'**il comprend au moins l'une des trois phases ouvertes de lecture correspondant aux séquences SEQ ID N°2, SEQ ID N°3 et SEQ ID N°4.

5. Polynucléotide isolé, naturel ou synthétique, répondant à l'une quelconque des séquences SEQ ID N°2, SEQ ID N°3 ou SEQ ID N°4.

6. Vecteur, **caractérisé en ce qu'**il comprend l'un des polynucléotides selon l'une quelconque des revendications 1 à 5.

7. Vecteur selon la revendication 6, **caractérisé en ce qu'**il s'agit d'un plasmide, d'un cosmide, d'un chromosome artificiel bactérien (BAC), d'un élément intégratif d'actinobactéries, d'un virus ou encore d'un bactériophage.

8. Utilisation de l'un au moins des polynucléotides selon l'une quelconque des revendications 1 à 5 ou de l'un de ses fragments d'au moins 15 nucléotides ou de l'un des vecteurs selon l'une quelconque des revendications 6 ou 7 comme sonde.

9. Utilisation de l'un au moins des polynucléotides selon l'une quelconque des revendications 1 à 5 ou de l'un de ses fragments d'au moins 15 nucléotides ou de l'un des vecteurs selon l'une quelconque des revendications 6 ou 7 comme amorce pour l'amplification de séquences nucléiques.

10. Polypeptide isolé ou synthétique, **caractérisé en ce qu'**il comprend au moins l'une quelconque des séquences SEQ ID N°7 à SEQ ID N°10.

11. Polypeptide isolé, naturel ou synthétique, **caractérisé en ce qu'**il correspond à l'une quelconque des séquences SEQ ID N°7 à SEQ ID N°10.

12. Polypeptide isolé, naturel ou synthétique, **caractérisé en ce qu'**il est codé par l'un des polynucléotides selon l'une quelconque des revendications 1 à 5 ou l'un des vecteurs selon l'une quelconque des revendications 6 ou 7.

13. Utilisation d'un polynucléotide selon l'une quelconque des revendications 1 à 5 ou d'un vecteur tel que décrit dans l'une quelconque des revendications 6 ou 7, pour la préparation d'un polypeptide tel que décrit dans l'une quelconque des revendications 10 à 12.

14. Utilisation, particulièrement *in vitro,* d'au moins un polypeptide selon l'une quelconque des revendications 10 à 12, seul ou en combinaison, pour la préparation de cyclodipeptides et/ou de dérivés des dicétopipérazines substituées en positions 3 et 6 par des chaînes latérales d'acides aminés α,β-insaturées, particulièrement de l'albonoursine.

15. Utilisation d'au moins un polynucléotide selon l'une quelconque des revendications 1 à 5 ou d'un vecteur tel que décrit dans l'une quelconque des revendications 6 ou 7, pour la préparation d'un système biologique modifié ou d'un système acellulaire *in vitro* modifié.

16. Utilisation selon la revendication 15, **caractérisée en ce que** le système biologique modifié est un microorganisme ou un système d'expression hétérologue utilisant comme hôtes des procaryotes ou des eucaryotes.

17. Système biologique modifié, **caractérisé en ce qu'**il contient au moins l'un des polynucléotides tels que décrits dans l'une quelconque des revendications 1 à 5, et/ou au moins l'un des vecteurs tels que décrits dans l'une quelconque des revendications 6 ou 7.

18. Système biologique selon la revendication 17, **caractérisé en ce qu'**il est constitué par un microorganisme ou un système d'expression hétérologue utilisant comme hôtes des procaryotes ou des eucaryotes, ou encore un système acellulaire *in vitro.*

19. Système biologique selon la revendication 18, **caractérisé en ce que** le microorganisme est une bactérie telle que *Escherichia coli* ou *Streptomyces lividans.*

20. Système acellulaire *in vitro* modifié, **caractérisé en ce qu'**il contient au moins l'un des polynucléotides tels que décrits dans l'une quelconque des revendications 1 à 5, et/ou au moins l'un des vecteurs tels que décrits dans l'une quelconque des revendications 6 ou 7.

21. Utilisation d'au moins un système biologique modifié selon l'une quelconque des revendications 17 à 19 ou d'un système acellulaire *in vitro* modifié selon la revendication 20, pour la préparation de cyclodipeptides et/ou de dérivés des dicétopipérazines substituées en positions 3 et 6 par des chaînes latérales d'acides aminés α,β-insaturés, particulièrement de l'albonoursine.

22. Procédé de synthèse *in vitro* de cyclodipeptides, **caractérisé en ce que** :
(1) on met en contact, dans des conditions convenables, deux acides aminés, identiques ou différents et le polypeptide AlbC (SEQ ID N°9) et
(2) on purifie le cyclodipeptide obtenu.

23. Procédé de synthèse *in vitro* d'un dérivé de dicétopipérazine α,β-insaturée, substitué en positions 3 et 6 par des chaînes latérales d'acides aminés, **caractérisé en ce que** :
(1) on met en contact, dans des conditions convenables, deux acides aminés, identiques ou différents et le polypeptide AlbC (SEQ ID N°9) et l'on purifie le cyclodipeptide obtenu et
(2) on met en contact le cyclodipeptide obtenu à l'étape (1) et AlbA (SEQ ID N°6), AlbB1 (SEQ ID N°7) et AlbB2 (SEQ ID N°8) et l'on purifie le dérivé de dicétopipérazines α,β-insaturé obtenu.

24. Procédé selon la revendication 23, **caractérisé en ce que** le procédé comprend en outre à l'étape (2) le polypeptide AlbD (SEQ ID N°10).

25. Procédé selon l'une quelconque des revendications 22 à 24, **caractérisé en ce que** la quantité de polypeptides est comprise entre 0,1 nM et 10 µM, de préférence entre 10 nM et 1 µM.

26. Procédé de synthèse d'un cyclodipeptide, **caractérisé en ce que** :
(1) on met en contact un système biologique comprenant au moins le polynucléotide albC (SEQ ID N°3), dans des conditions appropriées à la culture dudit système biologique choisi et
(2) on purifie le cyclodipeptide obtenu.

27. Procédé selon la revendication 26, **caractérisé en ce que** le système biologique comprend en outre le polynucléotide albD (SEQ ID N°4).

28. Procédé de synthèse d'un dérivé de dicétopipérazines substituées en positions 3 et 6 par des chaînes latérales d'acides aminés α,β-insaturées, **caractérisé en ce que** :
(1) on met en contact dans des conditions appropriées, un système biologique comprenant un polynucléotide comprenant au moins albA, albB, et albC (SEQ ID N°1 à 3), dans des conditions appropriées à la culture dudit système biologique choisi, et
(2) on purifie le dérivé de dicétopipérazines α,β-insaturé obtenu.

29. Procédé selon la revendication 28, **caractérisé en ce que** le système biologique comprend en outre le polynucléotide albD (SEQ ID N°4).

30. Procédé selon l'une quelconque des revendications 26 à 29, **caractérisé en ce que** le système biologique est un microorganisme comme une bactérie telle que *Escherichia coli* ou *Streptomyces lividans* ou tout système d'expression hétérologue connu utilisant comme hôtes des procaryotes ou des eucaryotes, voire un système acellulaire *in vitro.*

31. Procédé selon l'une quelconque des revendications 22 à 30, **caractérisé en ce que** la quantité d'acides aminés est comprise entre 0,1 mM et 100 mM, de préférence entre 1 mM et 10 mM.

## Claims

1. An isolated or synthetic polynucleotide **characterized in that** it comprises at least the three open reading frames corresponding to the sequences SEQ ID No.1, SEQ ID No.2 and SEQ ID No.3.

2. The polynucleotide as claimed in claim 1, **characterized in that** it also comprises the open reading frame corresponding to the sequence SEQ ID No.4.

3. An isolated, natural or synthetic polynucleotide **characterized in that** it corresponds to the sequence SEQ ID No.5.

4. An isolated, natural or synthetic polynucleotide **characterized in that** it comprises at least one of the three open reading frames corresponding to the sequences SEQ ID No.2, SEQ ID No.3 and SEQ ID No.4.

5. An isolated, natural or synthetic polynucleotide corresponding to any one of the sequences SEQ ID No.2, SEQ ID No.3 or SEQ ID No.4.

6. A vector, **characterized in that** it comprises one of the polynucleotides as claimed in any one of claims 1 to 5.

7. The vector as claimed in claim 6, **characterized in that** it is a plasmid, a cosmid, a bacterial artificial chromosome (BAC), an integrative element of actinobacteria, a virus or else a bacteriophage.

8. The use of at least one of the polynucleotides as claimed in any one of claims 1 to 5 or of one of its fragments of at least 15 nucleotides or of one of the vectors as claimed in either one of claims 6 or 7, as a probe.

9. The use of at least one of the polynucleotides as claimed in any one of claims 1 to 5 or of one of its fragments of at least 15 nucleotides or of one of the vectors as claimed in either one of claims 6 or 7, as a primer for amplifying nucleic acid sequences.

10. An isolated or synthetic polypolypeptide **characterized in that** it comprises at least any one of the sequences SEQ ID No.7 to SEQ ID No.10.

11. An isolated, natural or synthetic polypeptide **characterized in that** it corresponds to any one of the sequences SEQ ID No.7 to SEQ ID No.10.

12. An isolated, natural or synthetic polypeptide **characterized in that** it is encoded by one of the polynucleotides as claimed in any one of claims 1 to 5 or one of the vectors as claimed in either one of claims 6 or 7.

13. The use of a polynucleotide as claimed in any one of claims 1 to 5 or of a vector as described in either one of claims 6 or 7, for preparing a polypeptide as described in any one of claims 10 to 12.

14. The use, particularly *in vitro,* of at least one polypeptide as claimed in any one of claims 10 to 12, alone or in combination, for preparing cyclodipeptides and/or diketopiperazine derivatives substituted in the 3-and 6-positions with α,β-unsaturated amino acid side chains, particularly albonoursin.

15. The use of at least one polynucleotide as claimed in any one of claims 1 to 5 or of a vector as described in either one of claims 6 or 7, for preparing a modified biological system or a modified *in vitro* acellular system.

16. The use as claimed in claim 15, **characterized in that** the modified biological system is a microorganism or a heterologous expression system using prokaryotes or eukaryotes as hosts.

17. A modified biological system, **characterized in that** it contains at least one of the polynucleotides as described in any one of claims 1 to 5 and/or at least one of the vectors as described in either one of claims 6 or 7.

18. The biological system as claimed in claim 17, **characterized in that** it consists of a microorganism or a heterologous expression system using prokaryotes or eukaryotes as hosts, or else an *in vitro* acellular system.

19. The biological system as claimed in claim 18, **characterized in that** the microorganism is a bacterium such as *Escherichia coli* or *Streptomyces lividans.*

20. A modified *in vitro* acellular system, **characterized in that** it contains at least one of the polynucleotides as described in any one of claims 1 to 5 and/or at least one of the vectors as described in either one of claims 6 or 7.

21. The use of at least one modified biological system as claimed in any one of claims 17 to 19 or of a modified *in vitro* acellular system as claimed in claim 20, for preparing cyclodipeptides and/or diketopiperazine derivatives substituted in the 3- and 6-positions with α,β-unsaturated amino acid side chains, particularly albonoursin.

22. A method for synthesizing, *in vitro,* cyclodipeptides, **characterized in that**:
(1) two amino acids, which may be identical or different, are brought into contact, under suitable conditions, with the polypeptide AlbC (SEQ ID No.9), and
(2) the cyclodipeptide obtained is purified.

23. A method for synthesizing, *in vitro,* an α,β-unsaturated diketopiperazine derivative substituted in the 3- and 6-positions with amino acid side chains, **characterized in that**:
(1) two amino acids, which may be identical or different, are brought into contact, under suitable conditions, with the polypeptide AlbC (SEQ ID No.9) and the cyclodipeptide obtained is purified, and
(2) the cyclodipeptide obtained in step (1) is brought into contact with AlbA (SEQ ID No.6), AlbB1 (SEQ ID No.7) and AlbB2 (SEQ ID No.8) and the α,β-unsaturated diketopiperazine derivative obtained is purified.

24. The method as claimed in claim 23, **characterized in that** the method also comprises, in step (2), the polypeptide AlbD (SEQ ID No.10).

25. The method as claimed in any one of claims 22 to 24, **characterized in that** the amount of polypeptides is between 0.1 nM and 10 µM, preferably between 10 nM and 1 µM.

26. A method for synthesizing a cyclodipeptide, **characterized in that**:
(1) a biological system comprising at least the polynucleotide albC (SEQ ID No.3) is brought into contact under conditions suitable for culturing said chosen biological system, and
(2) the cyclodipeptide obtained is purified.

27. The method as claimed in claim 26, **characterized in that** the biological system also comprises the polynucleotide albD (SEQ ID No.4).

28. A method for synthesizing a diketopiperazine derivative substituted in the 3- and 6-positions with α,β-unsaturated amino acid side chains, **characterized in that**:
(1) a biological system comprising a polynucleotide comprising at least albA, albB and albC (SEQ ID Nos.1 to 3) is brought into contact under conditions suitable for culturing said chosen biological system, and
(2) the α,β-unsaturated diketopiperazine derivative obtained is purified.

29. The method as claimed in claim 28, **characterized in that** the biological system also comprises the polynucleotide albD (SEQ ID No.4).

30. The method as claimed in any one of claims 26 to 29, **characterized in that** the biological system is a microorganism, for instance a bacterium such as *Escherichia coli* or *Streptomyces lividans,* or any known heterologous expression system using prokaryotes or eukaryotes as hosts, or even an *in vitro* acellular system.

31. The method as claimed in any one of claims 22 to 30, **characterized in that** the amount of amino acids is between 0.1 mM and 100 mM, preferably between 1 mM and 10 mM.

## Patentansprüche

1. Isoliertes oder synthetisches Polynucleotid, **dadurch gekennzeichnet, dass** es mindestens die drei offenen Leserahmen umfasst, die den Sequenzen SEQ ID NO: 1, SEQ ID NO: 2 und SEQ ID NO: 3 entsprechen.

2. Polynucleotid gemäß Anspruch 1, **dadurch gekennzeichnet, dass** es außerdem den offenen Leserahmen entsprechend der Sequenz SEQ ID NO: 4 umfasst.

3. Isoliertes, natürliches oder synthetisches Polynucleotid, **dadurch gekennzeichnet, dass** es der Sequenz SEQ ID NO: 5 entspricht.

4. Isoliertes, natürliches oder synthetisches Polynucleotid, dadurch charakterisiert, dass es mindestens einen der drei offenen Leserahmen entsprechend der Sequenzen SEQ ID NO: 2, SEQ ID NO: 3 und SEQ ID NO: 4 umfasst.

5. Isoliertes, natürliches oder synthetisches Polynucleotid entsprechend einer der Sequenzen SEQ ID NO: 2, SEQ ID NO: 3 oder SEQ ID NO: 4.

6. Vektor, **dadurch gekennzeichnet, dass** er eines der Polynucleotide gemäß einem der Ansprüche 1 bis 5 umfasst.

7. Vektor gemäß Anspruch 6, **dadurch gekennzeichnet, dass** es sich um ein Plasmid, ein Kosmid, ein bakterielles artifizielles Chromosom (BAC), ein integratives Element der Actinomyzeten, um ein Virus oder außerdem um einen Bakteriophagen handelt.

8. Verwendung mindestens eines Polynucleotids gemäß einem der Ansprüche 1 bis 5 oder eines seiner Fragmente aus mindestens 15 Nucleotiden oder eines der Vektoren gemäß einem der Ansprüche 6 oder 7 als Sonde.

9. Verwendung mindestens eines Polynucleotids gemäß einem der Ansprüche 1 bis 5 oder einem seiner Fragmente aus mindestens 15 Nucleotiden oder eines Vektors gemäß einem der Ansprüche 6 oder 7 als Primer zur Amplifikation der Nucleinsäuresequenzen.

10. Isoliertes oder synthetisches Polypeptid, **dadurch gekennzeichnet, dass** es mindestens eine der Sequenzen SEQ ID NO: 7 bis SEQ ID NO: 10 umfasst.

11. Isoliertes, natürliches oder synthetisches Polypeptid, **dadurch gekennzeichnet, dass** es einer der Sequenzen SEQ ID NO: 7 bis SEQ ID NO: 10 entspricht.

12. Isoliertes, natürliches oder synthetisches Polypeptid, **dadurch gekennzeichnet, dass** es von einem der Polynucleotide gemäß einem der Ansprüche 1 bis 5, oder einem der Vektoren gemäß einem der Ansprüche 5 oder 7 codiert ist.

13. Verwendung eines Polynucleotids gemäß einem der Ansprüche 1 bis 5 oder eines Vektors wie in einem der Ansprüche 6 oder 7 beschrieben, zur Herstellung eines Polypeptids wie in einem der Ansprüche 10 bis 12 beschrieben.

14. Verwendung, besonders *in vitro,* mindestens eines Polypeptids gemäß einem der Ansprüche 10 bis 12, allein oder in Kombination, zur Herstellung von Cyclodipeptiden und/oder von Diketopiperazinderivaten an Position 3 und 6 mit α,β-ungesättigte Aminosäureseitenketten substituiert, besonders von Albonoursin.

15. Verwendung mindestens eines Polynucleotids gemäß einem der Ansprüche 1 bis 5 oder eines Vektors wie in einem der Ansprüche 6 oder 7 beschrieben, zur Herstellung eines modifizierten biologischen Systems oder eines in-vitro modifizierten azellulären Systems.

16. Verwendung gemäß Anspruch 15, **dadurch gekennzeichnet, dass** das modifizierte biologische System ein Mikroorganismus oder ein heterologes Expressionssystem ist, welches Prokaryonten oder Eukaryonten als Wirte benutzt.

17. Modifiziertes biologisches System, **dadurch gekennzeichnet, dass** es mindestens eines der Polynukleotide, wie beschrieben in einem der Ansprüche 1 bis 5, und/oder mindestens einen der Vektoren wie beschrieben in einem der Ansprüche 6 oder 7 enthält.

18. Biologisches System gemäß Anspruch 17, **dadurch gekennzeichnet, dass** es aus einem Mikroorganismus oder einem heterologen Expressionssystem, welches Prokaryonten oder Eukaryonten als Wirte benutzt, oder einem azellulären in-vitro-System besteht.

19. Biologisches System gemäß Anspruch 18, **dadurch gekennzeichnet, dass** der Mikroorganismus ein Bakterium wie *Escherichia coli* oder *Streptomyces lividans* ist.

20. In-vitro-modifiziertes azelluläres System, **dadurch gekennzeichnet, dass** es mindestens eines der Polynukleotide wie in einem der Ansprüche 1 bis 5 beschrieben, und/oder mindestens einen der Vektoren, wie in einem der Ansprüche 6 oder 7 beschrieben, enthält.

21. Verwendung mindestens eines modifizierten biologischen Systems gemäß einem der Ansprüche 17 bis 19 oder eines in-vitro-modifizierten azellulären Systems gemäß Anspruch 20 zur Herstellung von Cyclodipeptiden und/oder von Diketopiperazinderivaten, an den Positionen 3 und 6 substituiert mit α,β-ungesättigten Aminosäureketten, besonders von Albonoursin.

22. *In-vitro*-Syntheseverfahren für Cyclodipeptide, **gekennzeichnet durch**:
(1) Inkontaktbringen, unter angemessenen Bedingungen, zweier identischer oder unterschiedlicher Aminosäuren und dem Polypeptid AlbC (SEQ ID NO: 9) und
(2) Reinigen des erhaltenen Cyclodipeptids.

23. In-vitro-Syntheseverfahren für ein α,β-ungesättigtes Diketopiperazinderivat, an den Positionen 3 und 6 substituiert mit Aminosäureseitenketten, **gekennzeichnet durch**:
(1) Inkontaktbringen, unter angemessenen Bedingungen, zweier identischer oder unterschiedlicher Aminosäuren und dem Polypeptid AlbC (SEQ ID NO: 9) und Reinigen des erhaltenen Cyclodipeptids und
(2) Inkontaktbringen des Cyclodipeptids erhalten in Schritt (1) und AlbA (SEQ ID NO: 6), AlbB1 (SEQ ID NO: 7) und AlbB2 (SEQ ID NO: 8) und Reinigen des erhaltenen α,β-ungesättigten Diketopiperazinderivats.

24. Verfahren gemäß Anspruch 23, **dadurch gekennzeichnet, dass** das Verfahren im Schritt (2) außerdem das Polypeptid AlbD (SEQ ID NO: 10) umfasst.

25. Verfahren gemäß einem der Ansprüche 22 bis 24, **dadurch gekennzeichnet, dass** die Menge an Polypeptiden zwischen 0,1 nM und 10 µM ist, bevorzugt zwischen 10 nM und 1 µM.

26. Syntheseverfahren für ein Cyclodipeptid, **gekennzeichnet durch**:
(1) Inkontaktbringen eines biologischen Systems, das mindestens das Polynucleotid AlbC (SEQ ID NO: 3) beinhaltet, unter geeigneten Kulturbedingungen für das gewählte biologische System und
(2) Reinigen des erhaltenen Cyclodipeptids.

27. Verfahren gemäß Anspruch 26, **dadurch gekennzeichnet, dass** das biologische System außerdem das Polynucleotid AlbD (SEQ ID NO: 4) umfasst.

28. Syntheseverfahren für ein Diketopiperazinderivat an den Positionen 3 und 6 substituiert mit α,β-ungesättigten Aminosäureseitenketten, **gekennzeichnet durch**:
(1) Inkontaktbringen unter geeigneten Bedingungen, eines biologischen Systems, beinhaltend ein Polynucleotid, das mindestens AlbA, AlbB und AlbC (SEQ ID NO: 1 bis 3) beinhaltet, unter geeigneten Kulturbedingungen für das gewählte biologische System, und
(2) Reinigen des erhaltenen α,β-ungesättigten Diketopiperazinderivats.

29. Verfahren gemäß Anspruch 28, **dadurch gekennzeichnet, dass** das biologische System außerdem das Polynucleotid AlbD (SEQ ID NO: 4) beinhaltet.

30. Verfahren gemäß einem der Ansprüche 26 bis 29, **dadurch gekennzeichnet, dass** das biologische System ein Mikroorganismus, wie ein Bakterium, wie *Escherichia coli* oder *Streptomyces lividans* ist, oder jegliches bekanntes heterologes Expressionssystem, welches als Wirte Prokaryonten oder Eukaryonten benutzt, und sogar ein azelluläres *in-vitro-*System ist.

31. Verfahren gemäß einem der Ansprüche 22 bis 30, **dadurch gekennzeichnet, dass** die Menge an Aminosäuren zwischen 0,1 mM und 100 mM, bevorzugt zwischen 1 mM und 10 mM ist.


### LISTE DE SEQUENCES

<110> Commissariat à l'Energie Atomique Centre National de la Recherche Scientifique
   GONDRY Muriel
   GENET Roger
   LAUTRU Sylvie
   PERNODET Jean-Luc
<120> Polynucléotides et polypeptides codés par lesdits polynucléotides impliqués dans la synthèse de dérivés des dicétopipérazines
<130> CGA263/83FR
<140>
   <141>
<160> 23
<170> PatentIn Ver. 2.1
<210> 1
   <211> 657
   <212> ADN
   <213> Streptomyces noursei
<400> 1
   gtgaggcgcc acccatcgca ttcgccgtac cgcggcgggt gtgaggtgcg cccaaaaaga 60
   aggggattga tgttagctca cagttcatct gaatcgccgc cggaatcctt gccggacgcg 120
   tggacggtcc tcaaaacccg taccgccgtc cgcaattacg cgaaagagcc ggtcgacgac 180
   gcgctgatcg agcagctgtt ggaggccatg ctcgccgcgc cgaccgcctc caaccggcag 240
   gcgtggtcgt tcatggtggt gcgcaggccc gccgcggtcc gccggctgcg cgcgttctcg 300
   cccggggtgc tgggaacccc cgccttcttc gtcgtggcct gcgtcgaccg cagtctgacc 360
   gacaacctct ccccgaagct ctcgcagaag atctacgaca ccagcaagct ctgtgtcgcc 420
   atggcggtgg agaacctgct gctcgcggcg cacgcggccg gcctgggcgg atgcccggtg 480
   ggcagcttca ggtccgacat cgtcaccagc atgctcggta tcccggaaca catcgagccg 540
   atgctcgtgg tcccgatcgg ccgtcccgcg acagccctcg tcccctccca gcgccgcgcc 600
   aagaatgagg tcgtcaacta tgaatcctgg ggaaaccgtg ctgccgcccc aactgcg    657
<210> 2
   <211> 318
   <212> ADN
   <213> Streptomyces noursei
<400> 2
   atgaatcctg gggaaaccgt gctgccgccc caactgcgtg aggagatcgc gctcctcgcc 60
   gtctatctgc tcagcagcgg ccgcggactc ctggaggagc cggccgacta cggaatttac 120
   cgctgtaccg acggggcccg tcgggcgctc caactcctcg acgaacacgg cgggagcacg 180
   gcacggctga ccgccgtccg cgagcgtctc gacgaggtca tgttcgcgcc gatgggcgag 240
   gaccgggaca tgggcgcgat tctggacgac ctgtgtcgcc aaatggcaga cgctcttccg 300
   gaaattgaaa ccccctga                                              318
<210> 3
   <211> 720
   <212> ADN
   <213> Streptomyces noursei
<400> 3
   atgcttgcag gcttagttcc cgcgccggac cacggaatgc gggaagaaat acttggcgac 60
   cgcagccgat tgatccggca acgcggtgag cacgccctca tcggaatcag tgcgggcaac 120
   agttatttca gccagaagaa caccgtcatg ctgctgcaat gggccgggca gcgtttcgag 180
   cgcaccgatg tcgtctatgt cgacacccac atcgacgaga tgctgatcgc cgacggccgc 240
   agcgcgcagg aggccgagcg gtcggtcaaa cgcacgctca aggatctgcg gcgcagactc 300
   cggcgctcgc tggagagcgt gggcgaccac gccgagcggt tccgtgtccg gtccctgtcc 360
   gagctccagg agacccctga gtaccgggcc gtacgcgagc gcaccgaccg ggccttcgag 420
   gaggacgccg aattcgccac cgcctgcgag gacatggtgc gggccgtggt gatgaaccgg 480
   cccggtgacg gcgtcggcat ctccgcggaa cacctgcggg ccggtctgaa ctacgtgctg 540
   gccgaggccc cgctcttcgc ggactcgccc ggagtcttct ccgtcccctc ctcggtgctc 600
   tgctaccaca tcgacacccc gatcacggcg ttcctgtccc ggcgcgagac cggtttccgg 660
   gcggccgagg gacaggcgta cgtcgtcgtc aggccccagg agctggccga cgcggcctag 720
<210> 4
   <211> 834
   <212> ADN
   <213> Streptomyces noursei
<400> 4
   atgtcatggg gaggacagga cacttgctca tggtgcggaa cggggcccct cggcgaagct 60
   gaagacgtag gaagacagca cacgtcgcac gccgggggac ccgtcatgac tcaagccgcc 120
   accgtcaccg ccaccacgag ccagggcagg gcactcctgc ggagcctgac gccgctgttc 180
   gtggacgccg cgatcccgct cggctcgtac ttcctcctcg ccgagggctt cggcatgagc 240
   acggtcgccg cgctggcctg gagcagcgtg gtcccggcgc tgcgcacgat ctggggcctg 300
   gtccgggagc ggacggtcaa cggcctcgcg ctgctgatcc tcgtcgtcaa cgtggtgggg 360
   ctggcgacga gcaccctgac cggcgatgcc cggctgatga tggccaagga cagcggcgtc 420
   agcagcgtcg tcgggatcgc gatcctgctc tcggtgcgcg gccggcgccc gctgatgacc 480
   gccggactcc ggccctgggt gaccaaggga agcccggagg ggaacgccgc atgggaccgg 540
   ctgtgggcgc gcagcgcgcg gttccggcaa ctggagcggc gattctcgac ggtctggggg 600
   agcgccctgc tgatcgagtg cgtggtcaag gtcgtcggtg cgtacgtcct gccggtgcac 660
   accatggtgt ggctgggcac ggtgctgacg gtggtggcga tcctgctggc catggtggtc 720
   gcgggcggcg gcagcgccga gccgatggag cggatggtca aggccgaggt cggggccgcc 780
   ggcgaggccg ccacggcggg gaacgccgag ccggcgccgg ccgccgcggc ctga 834
<210> 5
   <211> 3839
   <212> ADN
   <213> Streptomyces noursei
<400> 5
   ggatccgtcc cgacgggcgg gaaccggtga ggcgccaccc atcgcattcg ccgtaccgcg 60
   gcgggtgtga ggtgcgccca aaaagaaggg gattgatgtt agctcacagt tcatctgaat 120
   cgccgccgga atccttgccg gacgcgtgga cggtcctcaa aacccgtacc gccgtccgca 180
   attacgcgaa agagccggtc gacgacgcgc tgatcgagca gctgttggag gccatgctcg 240
   ccgcgccgac cgcctccaac cggcaggcgt ggtcgttcat ggtggtgcgc aggcccgccg 300
   cggtccgccg gctgcgcgcg ttctcgcccg gggtgctggg aacccccgcc ttcttcgtcg 360
   tggcctgcgt cgaccgcagt ctgaccgaca acctctcccc gaagctctcg cagaagatct 420
   acgacaccag caagctctgt gtcgccatgg cggtggagaa cctgctgctc gcggcgcacg 480
   cggccggcct gggcggatgc ccggtgggca gcttcaggtc cgacatcgtc accagcatgc 540
   tcggtatccc ggaacacatc gagccgatgc tcgtggtccc gatcggccgt cccgcgacag 600
   ccctcgtccc ctcccagcgc cgcgccaaga atgaggtcgt caactatgaa tcctggggaa 660
   accgtgctgc cgccccaact gcgtgaggag atcgcgctcc tcgccgtcta tctgctcagc 720
   agcggccgcg gactcctgga ggagccggcc gactacggaa tttaccgctg taccgacggg 780
   gcccgtcggg cgctccaact cctcgacgaa cacggcggga gcacggcacg gctgaccgcc 840
   gtccgcgagc gtctcgacga ggtcatgttc gcgccgatgg gcgaggaccg ggacatgggc 900
   gcgattctgg acgacctgtg tcgccaaatg gcagacgctc ttccggaaat tgaaaccccc 960
   tgacggctgt ccggggcaac cccaaaagga cttcttagca tgcttgcagg cttagttccc 1020
   gcgccggacc acggaatgcg ggaagaaata cttggcgacc gcagccgatt gatccggcaa 1080
   cgcggtgagc acgccctcat cggaatcagt gcgggcaaca gttatttcag ccagaagaac 1140
   accgtcatgc tgctgcaatg ggccgggcag cgtttcgagc gcaccgatgt cgtctatgtc 1200
   gacacccaca tcgacgagat gctgatcgcc gacggccgca gcgcgcagga ggccgagcgg 1260
   tcggtcaaac gcacgctcaa ggatctgcgg cgcagactcc ggcgctcgct ggagagcgtg 1320
   ggcgaccacg ccgagcggtt ccgtgtccgg tccctgtccg agctccagga gacccctgag 1380
   taccgggccg tacgcgagcg caccgaccgg gccttcgagg aggacgccga attcgccacc 1440
   gcctgcgagg acatggtgcg ggccgtggtg atgaaccggc ccggtgacgg cgtcggcatc 1500
   tccgcggaac acctgcgggc cggtctgaac tacgtgctgg ccgaggcccc gctcttcgcg 1560
   gactcgcccg gagtcttctc cgtcccctcc tcggtgctct gctaccacat cgacaccccg 1620
   atcacggcgt tcctgtcccg gcgcgagacc ggtttccggg cggccgaggg acaggcgtac 1680
   gtcgtcgtca ggccccagga gctggccgac gcggcctagt tgggggcgtc cgcgggcgga 1740
   cctgcctccc cacccgctcc cggtgccggc gccgggcatg acaaatgtca tggggaggac 1800
   aggacacttg ctcatggtgc ggaacggggc ccctcggcga agctgaagac gtaggaagac 1860
   agcacacgtc gcacgccggg ggacccgtca tgactcaagc cgccaccgtc accgccacca 1920
   cgagccaggg cagggcactc ctgcggagcc tgacgccgct gttcgtggac gccgcgatcc 1980
   cgctcggctc gtacttcctc ctcgccgagg gcttcggcat gagcacggtc gccgcgctgg 2040
   cctggagcag cgtggtcccg gcgctgcgca cgatctgggg cctggtccgg gagcggacgg 2100
   tcaacggcct cgcgctgctg atcctcgtcg tcaacgtggt ggggctggcg acgagcaccc 2160
   tgaccggcga tgcccggctg atgatggcca aggacagcgg cgtcagcagc gtcgtcggga 2220
   tcgcgatcct gctctcggtg cgcggccggc gcccgctgat gaccgccgga ctccggccct 2280
   gggtgaccaa gggaagcccg gaggggaacg ccgcatggga ccggctgtgg gcgcgcagcg 2340
   cgcggttccg gcaactggag cggcgattct cgacggtctg ggggagcgcc ctgctgatcg 2400
   agtgcgtggt caaggtcgtc ggtgcgtacg tcctgccggt gcacaccatg gtgtggctgg 2460
   gcacggtgct gacggtggtg gcgatcctgc tggccatggt ggtcgcgggc ggcggcagcg 2520
   ccgagccgat ggagcggatg gtcaaggccg aggtcggggc cgccggcgag gccgccacgg 2580
   cggggaacgc cgagccggcg ccggccgccg cggcctgaga ccgcgcggcg ggggagttgg 2640
   ggaaatcgcg tacaggattg gcgcgtcgag cacccccgcc ctcgataggg cgggcccccg 2700
   gcgcatatgg tcggcgatgc gacgggacat cggagccccg cgtcgacggt tcaacggcga 2760
   tccggacggc acgcggcttt cgtcggccac gaagggaacg gaagtcatgt cgactgttca 2820
   cactggggtc acgcagagcg gtctcaccgc cgagctggcc tccctgcacg ccgagctcgt 2880
   ccgtcggaat cccggtgaag cggagttcca ccaggcggcc ctggaggtcc tcgaaacgct 2940
   ggcaccggtg ctcaccgccc ggccggagtt cgccgacgcc aaggtcctgg agcggatcgt 3000
   cgagccggag cggcagatca tgttccgcgt gccctggcag gacgactccg gcacgatccg 3060
   ggtcaaccgc ggcttccggg tggagttcaa cagcgcgctc ggcccctaca agggcggcct 3120
   gcggttccac gcgtccgtca acctcggcat cgtgaagttc ctcggcttcg agcagatctt 3180
   caagaacgcc ctgaccgggc tgaacatcgg cggcggcaag ggcggcagcg acttcgaccc 3240
   gcacggcagg tcggacgccg aggtgatgcg cttctgccag tccttcatga ccgagctgca 3300
   ccgtcacctg ggcgagcaca ccgacgtgcc ggctggcgac atcggcgtcg gcggccggga 3360
   gatcggctac ctcttcggcc agtaccggcg gatcaccaac cgctgggagg ccggcgtcct 3420
   gaccggcaag ggcctggcgt ggggcggctc caaggcccgt acggaggcca ccggttacgg 3480
   caatgtgctg ttcaccgagg agatgctcaa gcagcgcggc gaggagctgg acggccagca 3540
   ggtggtggtc tccgggtccg gcaacgtcgc catctacacc atcgagaagg cccaggcgct 3600
   cggcgccaac gtcctgaccg tctcggactc cggcggctac gtcgtcgacg agaagggcat 3660
   cgacctggcg ctgctcaagc aggtcaagga ggtcgagcgc ggccgggtcg gcgactacgc 3720
   ccagcggcgc ggcagttcgg cgaagtacgt cgccggcggg agcgtgtggg acgtcgcctg 3780
   tgacgtggcg ctgccgtcgg ccacccagaa cgagctcgac gcggacgccg cccggatcc  3839
<210> 6
   <211> 219
   <212> PRT
   <213> Streptomyces noursei
<400> 6
   Met Arg Arg His Pro Ser His Ser Pro Tyr Arg Gly Gly Cys Glu Val
    1               5                  10                  15
   Arg Pro Lys Arg Arg Gly Leu Met Leu Ala His Ser Ser Ser Glu Ser
               20                  25                  30
   Pro Pro Glu Ser Leu Pro Asp Ala Trp Thr Val Leu Lys Thr Arg Thr
           35                  40                  45
   Ala Val Arg Asn Tyr Ala Lys Glu Pro Val Asp Asp Ala Leu Ile Glu
       50                  55                  60
   Gln Leu Leu Glu Ala Met Leu Ala Ala Pro Thr Ala Ser Asn Arg Gln
   65                  70                  75                  80
   Ala Trp Ser Phe Met Val Val Arg Arg Pro Ala Ala Val Arg Arg Leu
                   85                  90                  95
   Arg Ala Phe Ser Pro Gly Val Leu Gly Thr Pro Ala Phe Phe Val Val
               100                 105                 110
   Ala Cys Val Asp Arg Ser Leu Thr Asp Asn Leu Ser Pro Lys Leu Ser
           115                 120                 125
   Gln Lys Ile Tyr Asp Thr Ser Lys Leu Cys Val Ala Met Ala Val Glu
       130                 135                 140
   Asn Leu Leu Leu Ala Ala His Ala Ala Gly Leu Gly Gly Cys Pro Val
   145                 150                 155                 160
   Gly Ser Phe Arg Ser Asp Ile Val Thr Ser Met Leu Gly Ile Pro Glu
                   165                 170                 175
   His Ile Glu Pro Met Leu Val Val Pro Ile Gly Arg Pro Ala Thr Ala
               180                 185                 190
   Leu Val Pro Ser Gln Arg Arg Ala Lys Asn Glu Val Val Asn Tyr Glu
           195                 200                 205
   Ser Trp Gly Asn Arg Ala Ala Ala Pro Thr Ala
       210                 215
<210> 7
   <211> 104
   <212> PRT
   <213> Streptomyces noursei
<400> 7
   Asn Pro Gly Glu Thr Val Leu Pro Pro Gln Leu Arg Glu Glu Ile Ala
    1               5                  10                  15
   Leu Leu Ala Val Tyr Leu Leu Ser Ser Gly Arg Gly Leu Leu Glu Glu
               20                  25                  30
   Pro Ala Asp Tyr Gly Ile Tyr Arg Cys Thr Asp Gly Ala Arg Arg Ala
           35                  40                  45
   Leu Gln Leu Leu Asp Glu His Gly Gly Ser Thr Ala Arg Leu Thr Ala
       50                  55                  60
   Val Arg Glu Arg Leu Asp Glu Val Met Phe Ala Pro Met Gly Glu Asp
   65                  70                  75                  80
   Arg Asp Met Gly Ala Ile Leu Asp Asp Leu Cys Arg Gln Met Ala Asp
                   85                  90                  95
   Ala Leu Pro Glu Ile Glu Thr Pro
               100
<210> 8
   <211> 99
   <212> PRT
   <213> Streptomyces noursei
<400> 8
   Met Leu Pro Pro Gln Leu Arg Glu Glu Ile Ala Leu Leu Ala Val Tyr
    1               5                  10                  15
   Leu Leu Ser Ser Gly Arg Gly Leu Leu Glu Glu Pro Ala Asp Tyr Gly
               20                  25                  30
   Ile Tyr Arg Cys Thr Asp Gly Ala Arg Arg Ala Leu Gln Leu Leu Asp
           35                  40                  45
   Glu His Gly Gly Ser Thr Ala Arg Leu Thr Ala Val Arg Glu Arg Leu
       50                  55                  60
   Asp Glu Val Met Phe Ala Pro Met Gly Glu Asp Arg Asp Met Gly Ala
   65                  70                  75                  80
   Ile Leu Asp Asp Leu Cys Arg Gln Met Ala Asp Ala Leu Pro Glu Ile
                   85                  90                  95
   Glu Thr Pro
<210> 9
   <211> 239
   <212> PRT
   <213> Streptomyces noursei
<400> 9
   Met Leu Ala Gly Leu Val Pro Ala Pro Asp His Gly Met Arg Glu Glu
   1               5                   10                  15
   Ile Leu Gly Asp Arg Ser Arg Leu Ile Arg Gln Arg Gly Glu His Ala
               20                  25                  30
   Leu Ile Gly Ile Ser Ala Gly Asn Ser Tyr Phe Ser Gln Lys Asn Thr
           35                  40                  45
   Val Met Leu Leu Gln Trp Ala Gly Gln Arg Phe Glu Arg Thr Asp Val
       50                  55                  60
   Val Tyr Val Asp Thr His Ile Asp Glu Met Leu Ile Ala Asp Gly Arg
   65                  70                  75                  80
   Ser Ala Gln Glu Ala Glu Arg Ser Val Lys Arg Thr Leu Lys Asp Leu
                   85                  90                  95
   Arg Arg Arg Leu Arg Arg Ser Leu Glu Ser Val Gly Asp His Ala Glu
               100                 105                 110
   Arg Phe Arg Val Arg Ser Leu Ser Glu Leu Gln Glu Thr Pro Glu Tyr
           115                 120                 125
   Arg Ala Val Arg Glu Arg Thr Asp Arg Ala Phe Glu Glu Asp Ala Glu
       130                 135                 140
   Phe Ala Thr Ala Cys Glu Asp Met Val Arg Ala Val Val Met Asn Arg
   145                 150                 155                 160
   Pro Gly Asp Gly Val Gly Ile Ser Ala Glu His Leu Arg Ala Gly Leu
                   165                 170                 175
   Asn Tyr Val Leu Ala Glu Ala Pro Leu Phe Ala Asp Ser Pro Gly Val
               180                 185                 190
   Phe Ser Val Pro Ser Ser Val Leu Cys Tyr His Ile Asp Thr Pro Ile
           195                 200                 205
   Thr Ala Phe Leu Ser Arg Arg Glu Thr Gly Phe Arg Ala Ala Glu Gly
       210                 215                 220
   Gln Ala Tyr Val Val Val Arg Pro Gln Glu Leu Ala Asp Ala Ala
   225                 230                 235
<210> 10
   <211> 277
   <212> PRT
   <213> Streptomyces noursei
<400> 10
   Met Ser Trp Gly Gly Gln Asp Thr Cys Ser Trp Cys Gly Thr Gly Pro
   1               5                   10                  15
   Leu Gly Glu Ala Glu Asp Val Gly Arg Gln His Thr Ser His Ala Gly
               20                  25                  30
   Gly Pro Val Met Thr Gln Ala Ala Thr Val Thr Ala Thr Thr Ser Gln
           35                  40                  45
   Gly Arg Ala Leu Leu Arg Ser Leu Thr Pro Leu Phe Val Asp Ala Ala
       50                  55                  60
   Ile Pro Leu Gly Ser Tyr Phe Leu Leu Ala Glu Gly Phe Gly Met Ser
   65                  70                  75                  80
   Thr Val Ala Ala Leu Ala Trp Ser Ser Val Val Pro Ala Leu Arg Thr
                   85                  90                  95
   Ile Trp Gly Leu Val Arg Glu Arg Thr Val Asn Gly Leu Ala Leu Leu
               100                 105                 110
   Ile Leu Val Val Asn Val Val Gly Leu Ala Thr Ser Thr Leu Thr Gly
           115                 120                 125
   Asp Ala Arg Leu Met Met Ala Lys Asp Ser Gly Val Ser Ser Val Val
       130                 135                 140
   Gly Ile Ala Ile Leu Leu Ser Val Arg Gly Arg Arg Pro Leu Met Thr
   145                 150                 155                 160
   Ala Gly Leu Arg Pro Trp Val Thr Lys Gly Ser Pro Glu Gly Asn Ala
                   165                 170                 175
   Ala Trp Asp Arg Leu Trp Ala Arg Ser Ala Arg Phe Arg Gln Leu Glu
               180                 185                 190
   Arg Arg Phe Ser Thr Val Trp Gly Ser Ala Leu Leu Ile Glu Cys Val
           195                 200                 205
   Val Lys Val Val Gly Ala Tyr Val Leu Pro Val His Thr Met Val Trp
       210                 215                 220
   Leu Gly Thr Val Leu Thr Val Val Ala Ile Leu Leu Ala Met Val Val
   225                 230                 235                 240
   Ala Gly Gly Gly Ser Ala Glu Pro Met Glu Arg Met Val Lys Ala Glu
                   245                 250                 255
   Val Gly Ala Ala Gly Glu Ala Ala Thr Ala Gly Asn Ala Glu Pro Ala
               260                 265                 270
   Pro Ala Ala Ala Ala
           275
<210> 11
   <211> 20
   <212> PRT
   <213> Streptomyces noursei
<400> 11
   Glu Pro Val Asp Asp Ala Leu Ile Glu Gln Leu Leu Glu Ala Met Leu
   1               5                   10                  15
   Ala Ala Pro Thr
               20
<210> 12
   <211> 12
   <212> PRT
   <213> Streptomyces noursei
<400> 12
   Asn Glu Val Val Asn Tyr Glu Xaa Trp Gly Asn Arg
   1               5                   10
<210> 13
   <211> 9
   <212> PRT
   <213> Streptomyces noursei
<400> 13
   Gln Ala Xaa Ser Phe Met Val Val Arg
   1               5
<210> 14
   <211> 17
   <212> ADN
   <213> Séquence artificielle
<220>
   <223> Description de la séquence artificielle:amorce
<400> 14
   garccsgtsg acgacgc 17
<210> 15
   <211> 17
   <212> ADN
   <213> Séquence artificielle
<220>
   <223> Description de la séquence artificielle: amorce
<400> 15
   gcgtcgtcsa csggytc 17
<210> 16
   <211> 20
   <212> ADN
   <213> Séquence artificielle
<220>
   <223> Description de la séquence artificielle:amorce
<400> 16
   aacgargtsg tsaactacga 20
<210> 17
   <211> 20
   <212> ADN
   <213> Séquence artificielle
<220>
   <223> Description de la séquence artificielle:amorce
<400> 17
   tcgtagttsa csacytcgtt 20
<210> 18
   <211> 20
   <212> ADN
   <213> Séquence artificielle
<220>
   <223> Description de la séquence artificielle:amorce
<400> 18
   caggcstggw ssttcatggt 20
<210> 19
   <211> 20
   <212> ADN
   <213> Séquence artificielle
<220>
   <223> Description de la séquence artificielle:amorce
<400> 19
   accatgaass wccasgcctg 20
<210> 20
   <211> 47
   <212> ADN
   <213> Séquence artificielle
<220>
   <223> Description de la séquence artificielle:amorce
<400> 20
   cggctgcagg agaagggagc ggacatatgc ttgcaggctt agttccc 47
<210> 21
   <211> 42
   <212> ADN
   <213> Séquence artificielle
<220>
   <223> Description de la séquence artificielle:amorce
<400> 21
   cggtcccgtg gatccaagct tctaggccgc gtcggccagc tc 42
<210> 22
   <211> 36
   <212> ADN
   <213> Séquence artificielle
<220>
   <223> Description de la séquence artificielle:amorce
<400> 22
   gagcgggatc ctgcagtgtc atggggagga caggac 36
<210> 23
   <211> 41
   <212> ADN
   <213> Séquence artificielle
<220>
   <223> Description de la séquence artificielle:amorce
<400> 23
   cgatcacgtg gatccaagct tgccaatcct gtacgcgatt t 41